# EUROPEAN PATENT SPECIFICATION

(11) **EP 2 233 504 B1**
(45) Date of publication and mention of the grant of the patent: **01.04.2015**
(21) Application number: 08865748.1
(22) Date of filing: 24.11.2008
(51) Int. Cl.: C07K 17/08, C07K 14/505, A61K 47/48, A61P 7/06

(54) **AN ERYTHROPOIETIN MIMETIC PEPTIDE DERIVATIVES AND ITS PHARMACEUTICAL SALT, THE PREPARATION AND USES THEREOF**
EIN ERYTHROPOIETIN-MIMETISCHES PEPTIDDERIVAT UND SEIN PHARMAZEUTISCHES SALZ, SEINE HERSTELLUNG UND SEINE ANWENDUNGEN
DÉRIVÉ DE PEPTIDE MIMÉTIQUE D'ÉRYTHROPOÏÉTINE ET SON SEL PHARMACEUTIQUE, PRÉPARATION ET UTILISATIONS

(30) Priority: 12.12.2007 CN 200710198751
(43) Date of publication of application: 29.09.2010
(73) Proprietor: Jiangsu Hansoh Pharmaceutical Co., Ltd., Lianyungang, Jiangsu 222047 (CN)
(72) Inventor: LÜ, Aifeng, Lianyungang Jiangsu 222047 (CN); SUN, Changan, Lianyungang Jiangsu 222047 (CN); JIANG, Tao, Lianyungang Jiangsu 222047 (CN); WU, Wentao, Lianyungang Jiangsu 222047 (CN); WANG, Yali, Lianyungang Jiangsu 222047 (CN)
(74) Representative: Hart-Davis, Jason
(86) International application number: PCT/CN2008/001909
(87) International publication number: WO 2009/079910

(56) References cited:
- WO-A1-96/40749
- WO-A2-2004/101606
- WO-A2-2006/060148
- WO-A2-2006/062685
- CN-A- 1 226 176
- CN-A- 1 338 463
- CN-A- 1 680 449
- CN-A- 1 820 024
- CN-A- 1 823 087
- CN-A- 1 823 088
- JOHNSON D L ET AL.: 'Amino-terminal dimerization of an erythropoietin mimetic peptide results in increased erythropoietic activity.' vol. 4, no. 12, 1997, ISSN 1074-5521 pages 939 - 950

## Description

### FIELD OF THE INVENTION

The present invention relates to an erythropoietin (EPO) mimetic peptide derivative and its pharmaceutical salts which bind to the EPO receptor and activate the EPO receptor or have the agonistic effect of EPO. Specifically, the present invention relates to an EPO mimetic peptide derivative, derivatives for use in a method of treatment of disorders characterized by low level of EPO or insufficient or defective red blood cell population as defined in the appended claims.

### BACKGROUND OF THE INVENTION

EPO is a glycoprotein hormone with a molecular weight of about 34 kD. EPO in the plasma is composed of 165 amino acids with high degree of glycosylation, wherein the major component of glycosyl is sialic acid. Based on carbohydrate content, naturally occuring EPO is divided into two types, namely α and β-type, wherein α-type contains 34% carbohydrate and β-type contains 26% carbohydrate. These two types have the same biological characteristics, antigenicity and clinical effect. The human EPO gene is located in chromosome 7 long-Area 22. Its cDNA was successfully cloned in 1985, and recombinant human EPO (rHuEPO) have been largely produced using gene recombinant technology and widely used in clinical. EPO has been biosynthesized using recombinant DNA technology (Egrie, JC,Strickland, TW, Lane, J etc. (986) Immunobiology (Immunobiol)72: 213-224), which is the product of cloned human EPO gene inserted into Chinese hamster ovary cells (CHO cells) and expressed therein. Naturally occurring human EPO is firstly translated into polypeptide chains containing 166 amino acids with position 166 being arginine. In the post-translational modifications arginine on position 166 is degraded using hydroxyl peptidase. The molecular weight of human EPO without glycosyl is 18236Da. In the intact EPO molecule, glycosyl accounts for about 40% of the total molecular weight (J.Biol.Chem.262: 12059).

EPO is the first cytokine applied in clinical and by far the best known hemoglobin-increasing preparation with single and safe effect. It has a certain effect for renal anemia, aplastic anemia, multiple myeloma, and paroxysmal nocturnal hematuria; in addition, the application of EPO can reduce the amount of blood transfusion in the surgery and to some extent cure anemia caused by malignant tumor, chemotherapy and rheumatoid arthritis. Since EPO is primarily generated by renal tubular endothelial cells, anemia caused by renal disease is the first indication of EPO; the curative efficacy of EPO on renal anemia is almost 100%, but EPO dose not improve renal function. Treatment with EPO is safe, effective and suitable for long-term treating; in addition, it addresses the problem of shortage of blood supply. On the markets of global biotech-drug in 2006, EPO recombinant drugs accounted for 11.9 billion U.S. dollars. There exists a huge market capacity.

As early as 1989, recombinant human EPO (EPOGEN) was approved by U.S. FDA for the treatment of renal anemia, but it is only since 1992 that EPOGEN entered Chinese market. The annual morbidity of chronic nephritis is about 0.25% in China, of which a considerable proportion of patients will eventually develop renal failure. The number of patients with renal anemia is about 500-600 thousands each year. According to conservative estimates of consumption together with consumption in other cancer-related anemia, the domestic market capacity is about 1.2-1.6 billion or even more (calculated with the current price being 30-40 CNY/dose, the average weight of patients being 50Kg). From the late 1990s, EPO has been ranked as best-selling drugs in China's major cities. The amount of consumption is 62.13 million yuan in sample hospitals of major cities all over China in 2003, ranking NO.56. The expense on EPO in sample hospitals of major cities all over China increased to 80.49 million CNY in 2004, with year-on-year rise of 30%.

As an endogenous hormone acting on the marrow hematopoietic cells to promote proliferation, differentiation and ultimate mature of erythroid progenitor cells, EPO plays an important role in regulating oxygen status of the body. In the early embryo stage, EPO is generated by the liver and then gradually shift to the kidney. EPO is mainly secreted by renal tubular interstitial cells after birth.

During induction of red progenitor cell differentiation by EPO, the globulin is induced, which allows cells to recruite more hemoglobin with iron synthesizing function, which can combine with oxygen in matured red blood cells; therfore, red blood cell and hemoglobin play an important role in supplying oxygen to the body. This course is caused by interaction between EPO and surface receptor of red progenitor cell.

When the body is in a healthy state, the tissue may obtain enough oxygen from already existed red blood cells. At this time the body's EPO concentration is very low. This low but normal concentration of EPO is sufficient to stimulate to promote generation of red blood cell, which is normally lost during aging.

When the level of oxygen transport by red blood cells in the circulation system is reduced and hypoxia appears, the amount of EPO in the body will increase. The body hypoxia state may be caused by the following reasons: excessive radiation, reduced oxygen intake due to high latitude or long term coma, various types of anemia and so on. As a response to hypoxia stress of the body, a higher level of EPO can stimulate the differentiation of red progenitor cell to enhance its ability to produce red blood cells. When the number of red blood cells in the body exceeds the need of the normal tissue, the level of EPO in the circulation system is reduced. It is precisely because EPO have a crucial role for the formation of red blood cells that these hormones have a very broad prospect for the treatment and diagnosis of blood disease characterized by low generation and defect of red blood cells. Recent studys provide the basis for predicting the effect of EPO therapy in a variety of diseases, disorders, and hematological abnormalities, these diseases including: the use of EPO in the treatment of anemia in patients with chronic renal failure (CRF), and administration of EPO to patients with AIDS and receiving chemotherapy (Danna, RP, Rudnick,SA,Abels,RI: edited by MB, Garnick, EPO in Clinical Applications-An International Perspective. Marcel Dekker; 1990:p301-324).

Part of the biological effects of EPO can be regulated by the internal role of surface receptors on the cell membrane. Previously, when studying EPO protein binding to cell surface using immature red blood cells isolated from mice spleen, it is found that this protein is composed by two polypeptides, and its molecular weight is approximately 85000∼100000KD (see Sawyer, et al. (1987) Proc. Natl. Acad. Sci.USA 84:3690-3694 for a more detailed description). The number of binding sites of EPO has also been calculated. Each cell membrane contains about 800 to 1000 sites. In these binding sites, about 300 binding sites have a Kd level of 90pM. The binding of the remaining binding sites is weak, being about 570pM. Some studies have shown that, from the response to EPO of red blood cells from the spleen of mice infected with the friend virus anemia strain, about 400 binding sites are identified, wherein some have a high level Kd of 100pM and some have a low level Kd of 800pM.

The subsequent work is that two types of EPO receptor are transcripbed by a single gene. Said gene has been cloned now. For example, the DNA sequences and peptide encoding sequences of the mouse and human EPO receptor have been described in WO90/08822. Current model shows that binding of EPO to EPO receptor leads to activation and dimerization of two EPO receptors. This dimerization further leads to the initiation of signaling.

The application of cloned gene of EPO is helpful in finding agonists and antagonists of these important receptors. The peptide which can to some extent act on the EPO receptor has been identified and described. Specially, a group of peptides containing the major peptide fragment have been identified, which can bind to EPO recptor and stimulate differentiation and proliferation of EPO cells. However, the EC50 of the peptide which can stimulate differentiation and proliferation of EPO cells is very low, ranging from 20nM to 250nM. Therefore, the clinical applications of these peptides are very limited. In order to overcome the deficiencies of existing technologies, the present invention provides EPO mimetic peptide derivative with better biological activity and higher bioavailability, as well as its pharmaceutical salts and their preparation method.

For example, WO2004/101606 and WO2006/062685 discuss peptide compounds that are agonists of the erythropoietin receptor and therapeutic applications to treat disorders associated with insufficient or defective red blood cell production.

### DESCRIPTION OF THE INVENTION

The present invention aims to provide a EPO mimetic peptide derivative with better biological activity and higher bioavailability, as well as its pharmaceutical salts and their preparation method.

The invention also aims to provide a pharmaceutical composition comprising the above-mentioned EPO mimetic peptide derivative and its pharmaceutical salts, for use in the treatment of disorders characterized by low level of EPO or insufficient or defective red blood cell population.

The invention discloses a EPO mimetic peptide derivative of general formula (I) and its pharmaceutical salts with *in vivo* biological activity,

R₁-R₂-(CH₂)ₙ₁-R₃-(CH₂)ₙ₂-R₄-R₅ **(I)**

wherin R₁, R₅ are as defined in the appended claims and wherein n₁, n₂ are integers independently selected from 0 ∼10; R₂, R₄ are selected from -CO or -CH₂; R₃ is selected from NCO(CH₂)n₄NHR₆, CHOCONH(CH₂)n₅NHR₆, or CHSCON(CH₂)n₅NHR₆; wherein n₄ is an integer selected from 2∼10, n₅ is an integer selected from 2∼10 integer, R₆ is selected from H or methoxy polyethylene glycol derivatives.

In this aspect, there are four preferred embodiments:
[1] n₁,n₂ are 2, R₂,R₄, are-CO, R₃ is CHOCONH(CH₂)ₙ₅NHR₆, n₅ is 2, R₆ is H or methoxy polyethylene glycol derivatives.
[2] n₁,n₂ are 1, R₂,R₄ are-CO, R₃ is NCO(CH₂)ₙ₄NHR₆, n₄ is 2, R₆ is H or methoxy polyethylene glycol derivatives.
[3] n₁,n₂ are 2, R₂,R₄ are -CH₂, R₃ is CHOCONH(CH₂)ₙ₅NHR₆, n₅ is 2, R₆ is H or methoxy polyethylene glycol derivatives.
[4] n₁,n₂ are 1, R₂,R₄ are -CH₂, R₃ is NCO(CH₂)ₙ₄NHR₆, n₄ is 2, R₆ is H or methoxy polyethylene glycol derivatives.

The above-mentioned four preferred embodiments are in parallel, not being including or progressive relationship.

The above-mentioned four preferred embodiments can be further optimized, R₆ is methoxy polyethylene glycol derivatives, most preferably R₆ is methoxy polyethylene glycol derivatives, wherein the molecular weight of methoxy polyethylene glycol derivatives is from 5,000 to 100,000 Daltons, the structure of methoxy polyethylene glycol derivatives is selected from the branched or linear type.

In this aspect, through further comprehensive optimization we can obtain the following four preferred embodiments:
[1] n₁, n₂ are 2, R₁, R₅ are selected from SEQ ID NO:1∼SEQ ID NO: 8, R₂, R₄ are selected from-CO, -CH₂, R₃ is CHOCONH (CH₂) n₅NHR₆, wherein n₅ is selected from 2∼10, preferably 2; R₆ is a methoxy polyethylene glycol derivative with linear structure and molecular weight of 20,000 daltons.
[2] n₁, n₂ are 1, R₁, R₅ are selected from SEQ ID NO: 1∼SEQ ID NO: 8, R₂, R₄ are selected from-CO, -CH₂, R₃ is NCO(CH₂)ₙ₄NHR₆, wherein n₄ is selected from 2∼10 and preferably 2; R₆ is a methoxy polyethylene glycol derivative with linear structure and molecular weight of 20,000 daltons.
[3] n₁, n₂ are 2, R₁, R₅ are selected from SEQ ID NO: 1∼SEQ ID NO: 8, R₂, R₄ are selected from-CO, -CH₂, R₃ is CHOCONH(CH₂)ₙ₅NHR₆, wherein n₅ is selected from 2∼10 and preferably 2; R₆ is a methoxy polyethylene glycol derivative with linear structure and molecular weight of 40,000 daltons.
[4] n₁, n₂ are 1, R₁, R₅ are selected from SEQ ID NO: 1∼SEQ ID NO: 8, R₂, R₄ are selected from-CO, -CH₂, R₃ is NCO(CH₂)ₙ₄NHR₆, wherein n₄ is selected from 2∼10 and preferably 2; R₆ is a methoxy polyethylene glycol derivative with linear structure and molecular weight of 40,000 daltons.

The structure of the most preferred EPO mimetic peptide derivatives and their pharmaceutical salt is selected from:

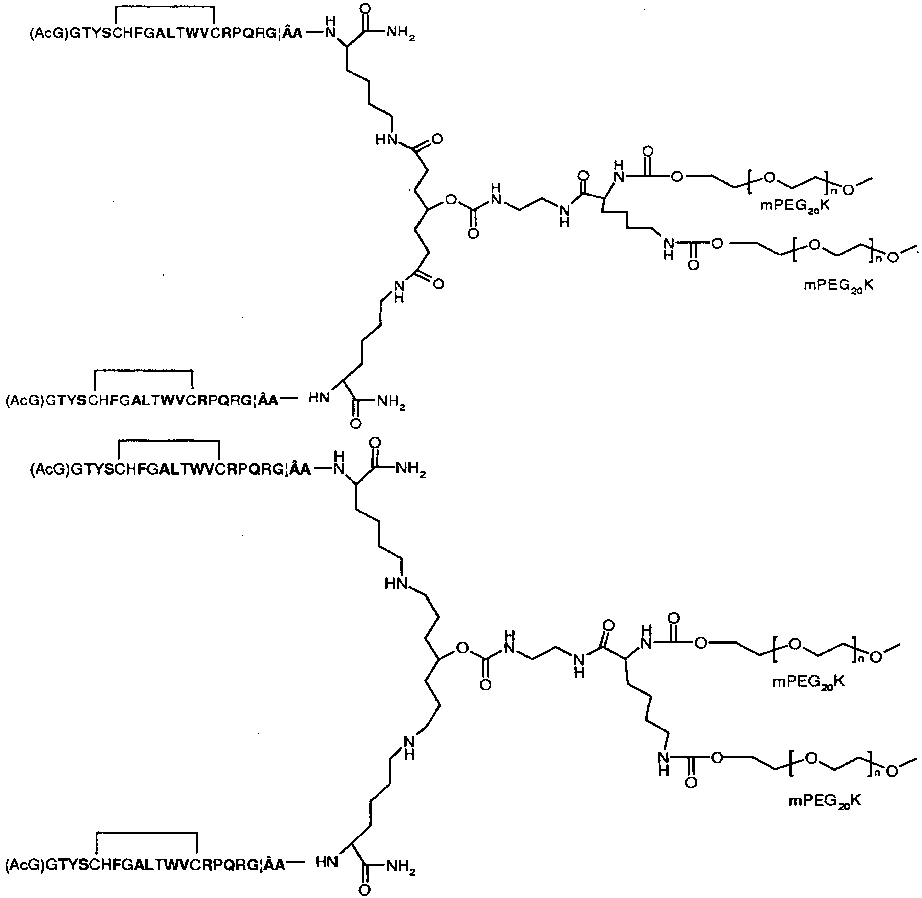

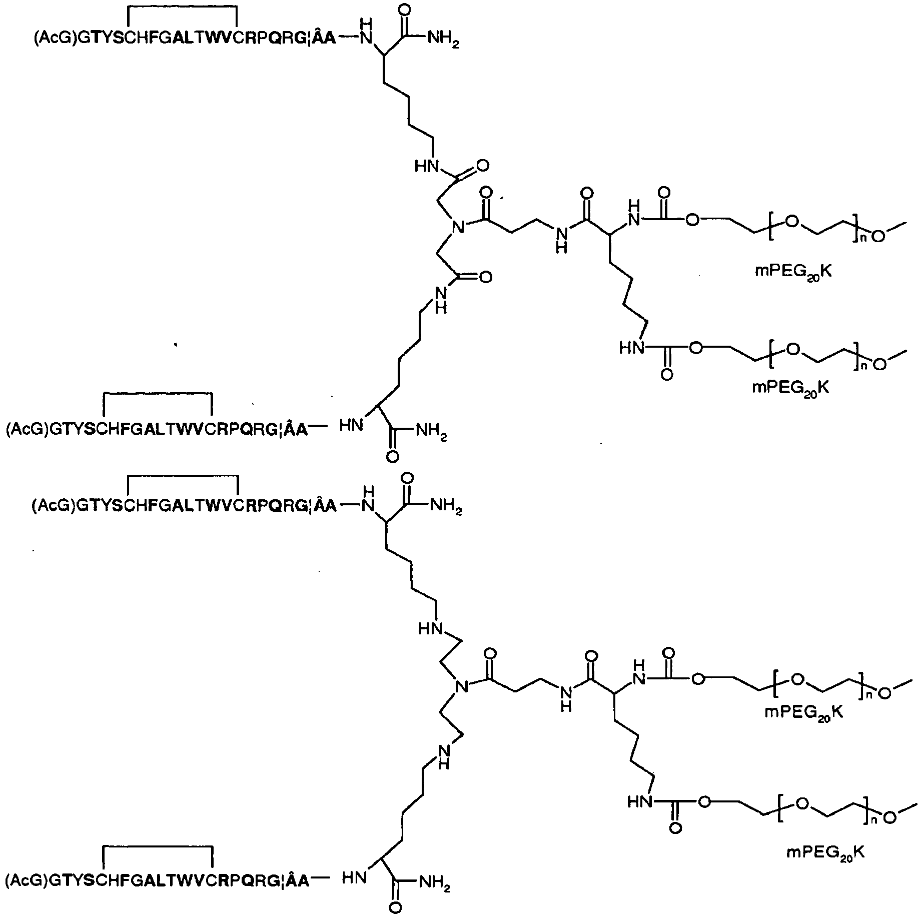

The EPO mimetic peptide derivatives provided herein are amphiphilic compounds, which can form salts by reacting with acidic or alkaline compounds through commonly known technology by one skilled in the art. Commonly used acids are selected from hydrochloric acid, hydrobromic acid, hydroiodic acid, sulfuric acid, phosphoric acid, p-toluenesulfonic acid, methanesulfonic acid, oxalic acid, p-bromophenyl sulfonic acid, carbonic acid, succinic acid, citric acid, benzoic acid, acetic acid; formed salts including sulfate, pyrophosphate, triflutate, sulfite, bisulfite, phosphate, biphosphate, dihydricphosphate, metaphosphate, pyrophosphate, chloride, bromide, iodide, acetate, propionate, caprylate, acrylate, formate, isobutyrate, caproate, enanthate, propiolate, oxalate, malonate, succinate, suberate, fumarate, maleate, butyne-1,4-dioate, hexyne-1,6-dioate, benzoate, chlorine benzoate, methyl benzoate, dinitrobenzoate, hydroxybenzoate, methoxybenzoate, phenylacetate, phenpropionate, phenylbutyrate, citrate, lactate, γ-hydroxybutyrate, glycolate, tartrate, methanesulfonate, propylsulfonate, naphtalin-1-sulfonate, naphtalin-2-sulfonate, mandelate and the like, preferably triflutate.

Alkaline substances can also react with EPO mimetic peptide derivatives to generate salts These alkaline substances are selected from ammonium, hydroxides of alkali metal or alkaline earth metal, as well as carbonate, bicarbonate, typically selected from sodium hydroxide, potassium hydroxide, ammonium hydroxide, sodium carbonate, potassium carbonate and so on.

The invention also discloses the preparation method of the above-mentioned EPO mimetic peptide derivatives and their pharmaceutical salts, as defined in the appended claims.

The invention also relates to a pharmaceutical composition, comprising:
(1) therapeutic amounts of the above-mentioned EPO mimetic peptide derivatives and their pharmaceutical salts of the general formula (I);
(2) pharmaceutical acceptable drug carrier.

The invention also discloses use of the drugs, namely using any of said mimetic peptide derivatives and its pharmaceutical salts of the invention in the preparation of a medicament for the treatment of disorders characterized by low level of EPO or insufficient or defective red blood cell group, especially for treatment of the following diseases: advanced renal failure or dialysis; AIDS-related anemia, autoimmune diseases or malignant tumor; cystic fibrosis; early premature anemia; chronic inflammatory disease-related anemia; spinal cord injury; acute blood loss; aging and cancer with abnormal production of red blood cells.

EPO mimetic peptide derivatives and their pharmaceutical salts provided in the present invention are capable of significantly promoting increase of the mouse peripheral blood reticulocyte counts, indicating that they stimulate erythropoiesis; at the same time they are also capable of greatly extending the half-life of the conjugates in the body. EPO mimetic peptide derivatives and the EPO protein have no significant influence on mature red blood cells, blood cell hematocrit, hemoglobin content, and also have no significant influence on the peripheral white blood cell count.

Solid phase synthesis is used to synthesize EPO mimic peptide monomers, whose basic principle is: firstly connect hydroxyl of hydroxyl-terminal amino acid of peptide chain to be synthesized with an insoluble polymer resin via covalent bond. Then amino acids attached to the solid phase carrier is used as the amino component to extend the polypeptide chain through removing amino protection group and reacting with excessive active carboxyl component. Repeat (condensation → washing →deprotection→ washing → the next round of condensation) operation to achieve the desired synthetic peptide chain length. Finally peptide chain is removed from the resin. After a purification treatment, the desired peptide is produced. The middle control of the reaction steps of condensation and deprotection exploys Ninhydrin detection method, namely when resin peptide chain has free amino, a blue color will appear after staining by the ninhydrin reagent. When there is no free amino, no color reaction will be developed (Ninhydrin reagent itself being yellow). Therefore, after carrying out the condensation reaction and detecting by ninhydrin, if yellow color is present (the colour of ninhydrin reagent itself), it means that this coupling step is completed and deprotection operation before coupling of the next amino acid can be carried out. If blue color is present, it means that there are still some free amino on the peptide chain. It needs further repeated coupling or change the current condensing agent until resin-peptide present yellow color after detection by ninhydrin.

The method of cyclization of monomer peptide is well-known by one skilled in the art. Cyclization of disulfide bonds is mainly through oxidization of the sulfhydryl in the side chain of the amino acid of the monomer peptide into disulfide bonds by oxidant. Specifically, monomer peptides are placed in DMSO solution or 5% amine bicarbonate solution to be auto-oxidized, or added to acetic acid solution containing I₂ to be oxidized, preferably added to acetic acid solution containing I₂ to be oxidized. Cyclization of amide bond is mainly through the formation of amide bond between carboxyl goup and amino group of amino side chain of monomer peptide in presence of condensing agent. The condensing agent added is well-known by one skilled in the art, usually including DIC, EDC, HATU, Pybop etc.

Synthesis of dimeric peptide is mainly through formation of -NH-CH2-bond or-NH-CO-bond between amino of side-chain of amino acid residue of EPO mimetic peptide monomer and functional small molecule. One skilled in the art can easily synthesize functional small molecule and connect it with monomer peptide cyclic peptide through known technology.

Dimeric peptide and active methoxy polyethylene glycol derivative are derived. The reaction system can be selected from organic solvent or available buffer system. When PEGylation reaction of the dimeric peptide is carried out in organic solvent, the following alkalis can be added in appropriate amout, including but not limited to, such as triethylamine, diisopropyl ethylamine, pyridine, 2,4,6-trimethyl pyridine. When the polyethylene glycol derivatization reaction is carried out in the buffer system, the buffer system can be selected from a variety of known available buffer, preferably pH 7.7 phosphate buffer.

The biological activity of EPO or EPO mimetic peptide derivatives and their pharmaceutical salts provided by this invention can be determined by various assay known in this field. Test of *in vivo* activity is performed as follows: mice are subcutaneously injected with EPO and EPO mimetic peptide derivatives and their pharmaceutical salts provided by this invention on three consecutive days. The mice are then sacrificed. The whole blood is takedn to carry out peripheral blood cells and reticulocyte count. The blood cell count is performed by automatic blood cell counter. Pharmacodynamic study is carried out by intravenous administration to Macaques with a dose of 1.35 mg/kg. The dose of EPO protein as a control drug is 240µ/Kg. The drugs are administered three times per week and continues for six weeks. The blood samples are collected to carry out related hematological index analysis.

### Summary of abbreviations used in the invention:

| Abbreviations | English name | Structure |
|---|---|---|
| Om | L-Ornithine | 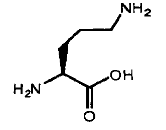 |
| Hoc | L-Homocysteine | 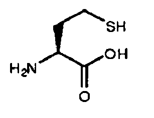 |
| DIC | N,N'-Diisopropylcarbodii mide | 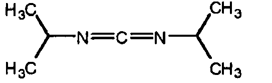 |
| EDC | 1-Ethyl-3-(3-dimethyllam inopropyl) carbodiimide hydrochloride | 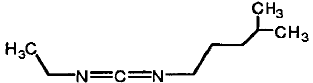 |
| PyBOP | Benzotriazol-1-yl-oxytrip yrrolidinophosphonium hexafluorophosphate | 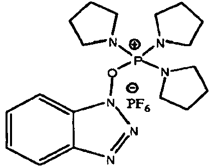 |
| HATU | 2-(1H-7-Azabenzotriazol-1-yl)-1,1,3,3-tetramethyl uronium hexafluorophosphate Methanaminium | 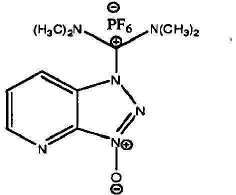 |
| DMAP | 4-Dimethylaminopyridine | 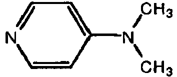 |
| Nle | norleucine | 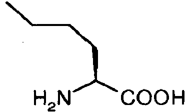 |
| mPEG₂-OSU(40k) | Branched Methoxy polyethylene glycol N-Hydroxysuccinimide(40k) | 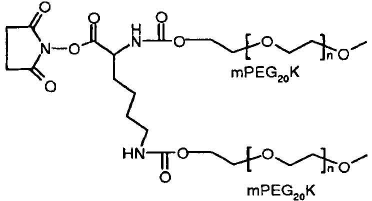 |

### Description of the Figures

Fig1 shows the influence of EPO mimetic peptide derivatives (HH-EPO-018) on Macaques hematocrit.
Fig2 shows the influence of EPO mimetic peptide derivatives (HH-EPO-018) on Macaques hemoglobin.

### PREFERRED EMBODIMENTS

For a more detailed description of the present invention, the following examples are proved.

### Example 1: synthesis of EPO mimetic peptide monomer peptide

Synthesis of EPO mimetic peptide monomer peptide is performed by a solid-phase peptide synthesis method. This peptide synthesis method has been reported in many literatures, see stewart, J.M., and Young, J.D., solid phase peptide synthesis 2d edition, novabiochem peptide synthesis notes. EPO mimetic peptide derivative monomer peptide provided by this invention is performed by manual synthesis methods. The resin is rink amind resin. The α-amino of the amino acid derivatives are protected by Fmoc (fluorene-formyl carbonyl). The thiol group of cysteine side chain, the amino group of glutamine side chain, and the imidazole group of histidine side chain are protected by Trt (trityl). The guanidine group of arginine side-chain is protected by Pbf (2,2,4,6,7-Pentamethyldihydrobenzofuran-5-sulfonyl). The indole group of tryptophan side chain, the amino group of the lysine side chain are protected by Boc (tert-Butoxycarbonyl). The hydroxyl group of the threonine side chain, the phenol group of the tyrosine side chain, the hydroxyl group of the serine side chain are protected by tBu (tert-butyl). The carboxyl of the C-terminal of the peptide chain of the EPO mimetic peptide derivative monomer peptide to be synthesized is attached to insoluble resin (rink amind resin) by covalent bond. Then amino acids attached to the solid phase carrier is used as the amino component to extend the polypeptide chain after removing amino protection groups by 20% piperidine / DMF solution and reacting with excessive amino acid derivative. Repeat (condensation → washing →deprotection→ washing → the next round of condensation) operation to achieve the desired synthetic peptide chain length. Finally peptide chain is removed from the resin using a mixed solution of trifluoroacetic acid, water, ethylene mercaptan, 3-isopropyl silane (92.5: 2.5: 2.5: 2.5). After ether sedimentation, the crude monomer peptide of EPO mimetic peptide derivative is obtained. The crude monomer peptide is separated and purified by C18 reverse-phase preparative column. Then EPO mimetic peptide derivative monomer peptide is obtained. The middle control of the reaction steps of condensation and deprotection exploys Ninhydrin detection method, namely when resin peptide chain has free amino, a blue color will appear after staining by the ninhydrin reagent. When there is no free amino, no color reaction will be developed (Ninhydrin reagent itself being yellow). Therefore, after carrying out the condensation reaction and detecting by ninhydrin, if yellow color is present (the colour of ninhydrin reagent itself), it means that this coupling step is completed and deprotection operation before coupling of the next amino acid can be carried out. If blue color is present, it means that there are still some free amino on the peptide chain. It needs further repeated coupling or change the current condensing agent until resin-peptide present yellow color after detection by ninhydrin.

### Example 2: Preparation of functional small molecules (LG-1)

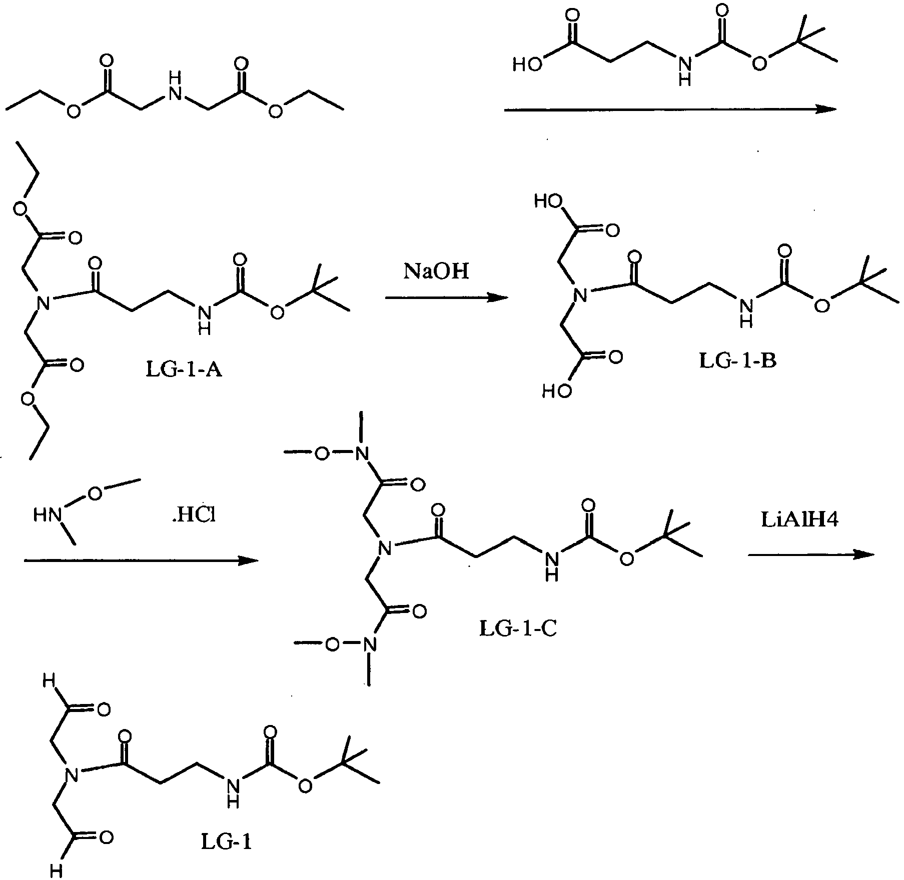

### Step 1: preparation of LG-1-A

Dissolve iminodiacetic acid diethyl ester (10.0g, 52.8mmol), Boc-β-alanine (10.0g, 52.8mmol) in 100mL dichloromethane, add DIC (8.0mL, 52.8mmol), stir it at room temperature overnight, filter the reaction solution, wash the filtrate with 100ml saturated NaHCO3, 50ml of 0.5N HCl solution, 100ml saturated saline in turn, seperate the organic layer, dry the organic layer with anhydrous MgSO₄. Filter the organic layer, concentrate it, and obtain colorless oily substanceliquid LG-1-A: 17g.

### Step 2: preparation of LG-1-B

Dissolve 17g LG-1-A in 100mL MeOH: THF = 1:1 mixture, and then add 25mL water, 5g NaOH (125mmol). Adjust pH value to 1 with 6N HCl solution after 2h stirring at room temperature. Extract the reaction solution with ethyl acetate four times. The organic layer is washed with saline, dried by anhydrous magnesium sulfate, concentrated at reduced pressure to produce a white semi-solid. Dissolve the products in 50mL dichloromethane, and add 300mL of n-hexane. The solution is a white paste. After concentration at reduced presure a white solid LG-1-B: 14g is obtained (Yield is about 90%).

### Step 3:Preparation of LG-1-C

Dissolve 7g LG-1-B (23mmol) in 80ml tetrahydrofuran, and with stirring add 4.6g N, N-methoxy-methyl-amine hydrochloride (46mmol) and 5.1g triethylamine (51mmol), and then add 4.4g DIC (32mmol), 4.7g HOBT (32mmol). Stir reaction overnight at room temperature. The next day, the reaction liquid is added into the water and extracted by 350ml ethyl acetate. The organic layer is washed by 200ml 2N HCl aqueous solution, 200ml saturated sodium bicarbonate solution, 100ml saturated saline in turn. The organic layer is separated and dried with anhydrous magnesium sulfate for 2 hours and then filtrated. The filtrate becomes oily substance after concentration at reduced pressure. After column chromatography, the target product LG-1-C is collected: 4.2 g, yield: 70%.

### Step 4:Preparation of LG-1

Dissolve 4.0g LG-1-C (10.2mmol) in 60ml tetrahydrofuran, cool it to 0 □ by ice-salt bath. Add LiAlH4 (340mg, 8.9mmol). After reaction at 0 □ for 30 minutes, add 4ml water, 4ml of 15% NaOH solution in turn. Filter the reaction solution. The filtrate is washed with tetrahydrofuran. After concentration to dry and silica gel column chromatography, LG-1 is obtained: 1.63g (6mmol, Yield: 58.8%)

### Example 3: Preparation of functional small molecules (LG-2)

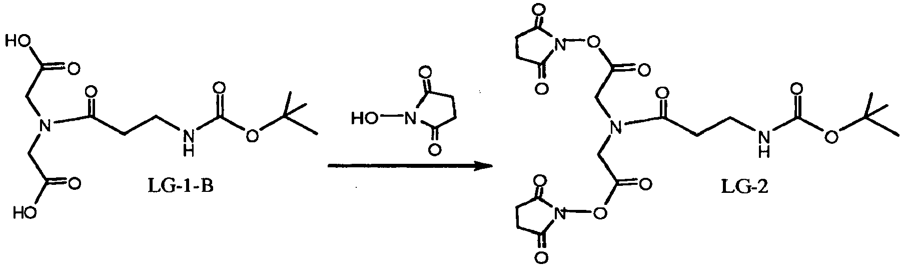

Dissolve 4g LG-1-B (13mmol) in 100mL N,N-dimethylformamide, and add hydroxysuccinimide (3.1g, 21mmol), DIC (4mL, 26mmol) and DMAP (4-dimethylamino pyridine) (12mg, 0.08mmol). After stirring overnight, the reaction solution is concentrated at reduced pressure. Dissolve the residue in 80ml ethyl acetate. The insoluble substances were filtered off. The organic phase is washed in turn with 40ml saturated sodium bicarbonate solution, 40ml saturated saline, 40ml of 0.5N HCl solution, 40ml saturated saline for one time. The organic layer is separated and dried by anhydrous magnesium sulfate. Filter the organic layer. The filtrate is concentrated at reduced pressure. A white solid LG-2 is obtained: 4.4g (Yield is about 68%).

### Example 4: Preparation of functional small molecules (LG-3)

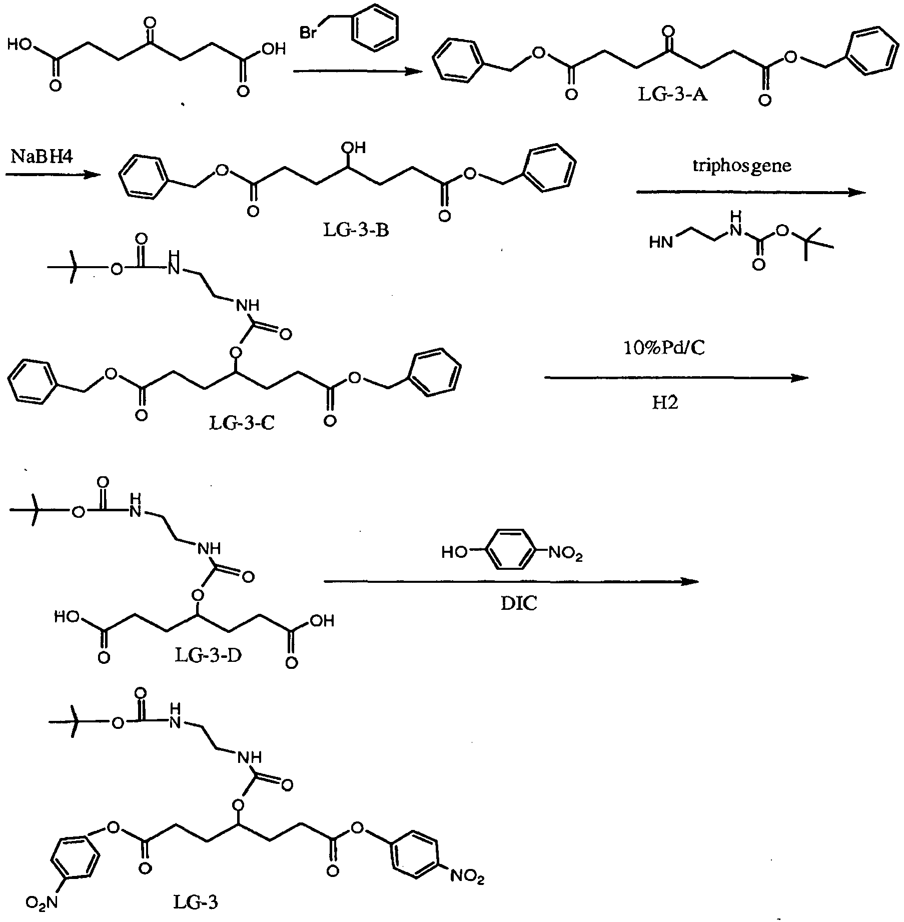

### Step 1: Preparation of LG-3-A

Dissolve 7.0g pentanone pimelic acid (0.04mol) in 100ml methanol. Add 5% CsCO3 methanol solution with stirring, and control the adding amount to make the pH of the reaction solution about 8.5 (determined by exact pH test paper). Stir it for 30 minutes after completing addition. Then filter the reaction solution. The filtrate becomes oily substanceafter after vacuum concentration. Dissolve the oily substance in about 100ml DMSO and warm up to 60□. Add 14g (0.08mol) benzyl bromide. After reaction for 8 hours, filter the reaction solution, wash the solid with a small amount of ether. Add 400ml ether into mother liquor, wash it with 200ml saturated saline. Separate the organic layer and dry it with anhydrous magnesium sulfate for 2 hours. Then filtrate it. When the filtrate is concentrated at reduced pressure to 1/5 of its original volume, it is placed in -20□ freezer overnight to crystallize. The next day, filter out the solids, dry, and obtain a white solid LG-3-A: 10.5g (Yield 74%).

### Step 2: Preparation of LG-3-B

Dissolve 2g LG-3-A (0.0056mol) in 20ml tetrahydrofuran. Keep the inside temperature of the solution below -10□, stir it and add 626mg NaBH4 (0.0168mol). After reaction for 1 h, add 200ml chilled ether, followed by adding 150ml saturated sodium bicarbonate solution to terminate the reaction. Keep rest to allow delaminate. The organic layer is washed for one time with saturated saline, and then dried with anhydrous Na₂SO₄ for 2 hours and filtered. The filtrate is concentrated at reduced pressure to produce LG-3-B: 1.9g (Yield: 94.6%).

### Step 3: Preparation of LG-3-C

Dissolve 3.2g LG-3-B (0.009mol) in 50ml dichloromethane. Add 4.34g triethylamine (0.043mol) below 0□ with stirring. Dissolve 1.33g (0.0045mol) triphosgene in 25ml dichloromethane. Then add it into the above solution drop by drop. Add 2.8g tert-Butoxycarbonyl-ethylenediamine after 1 hour. After reaction for 3h, the reaction mixture is adjusted to neutral with glacial acetic acid, and at this time a precipitate is generated. Filter off the precipitate. The filtrate is concentrated at reduced pressure and then dissolved in ether. Then washed with water for three times, with saturated saline for one time. The organic layer is separated out and dried with anhydrous magnesium sulfate for 2 hours and then filtrated. The filtrate is concentrated at reduced pressure to be oily. The oily substanceis is purified by silica gel column chromatography (mobile phase: petroleum ether: ethyl acetate = 10: 1). The target product is combined and collected, and is concentrated to produce a white solid LG-3-C: 1.5g (Yield 38.8%).

### Step 4: Preparation of LG-3-D

Dissolve 13g LG-3-C (0.031mol) in 8ml methanol and add about 200mg 10% Pd-C with stirring. After reaction under H2 at atmospheric pressure for 4h, filter off the activated carbon, and the filtrate is concentrated to produce the oily substance LG-3-D: 8.28g (Yield: 96.7%).

### Step 5: Preparation of LG-3

Dissolve 5g LG-3-D (0.018mol) in 10ml tetrahydrofuran. Add 4.7g of p-nitrophenol (0.043mol), and add DIC 4.2g (0.043) solution with stirring. Stir the reaction overnight. The next day, filter out the resulting precipitate, wash the filter cake with a small amount of ethyl acetate, dry the filtrate by concentration at reduced pressure. Add 100ml of ethyl acetate into the residue and make it dissolved. Wash it with 50 ml saturated saline for one time. Separate the organic layer and dry it with anhydrous magnesium sulfate for 2 hours. Then filter it. Concentrate the filtrate at reduced pressure to produce oily substance. Oily substance is purified by silica gel column chromatography (eluant: hexane/ethyl acetate 20:1 = 10:1). The target product is combined and collected, and concentrated at reduced pressure to dry to produce a white solid LG-3: 3.5g (Yield: 32%).

### Example 5: Preparation of functional small molecular (LG-4)

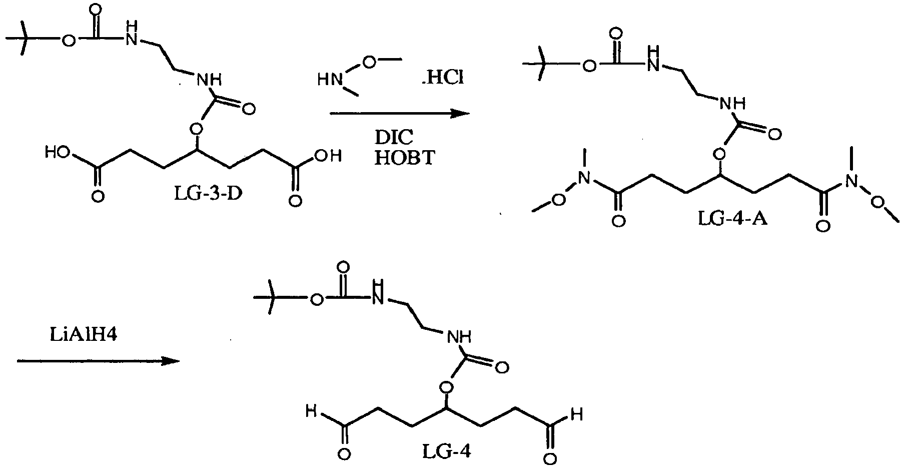

### Step 1: Preparation of LG-4-A

Dissolve 5g LG-3-D (0.018mol) in 60ml tetrahydrofuran. Add 3.51g N,N-methoxy-methyl-amine hydrochloride (0.036mol) and 4.0g triethylamine (0.04mol) with stirring. Then add 3.4g DIC (0.027mol) and 3.65g HOBT (0.027mol). Stir the reaction overnight at room temperature. The next day, add the reaction solution into 200ml water, and extract it with ethyl acetate twice, each time 200ml. Combine organic layer. Wash it in turn with 50ml 2N HCl solution, 100ml saturated NaHCO3 solution, 100ml saturated saline for one time. Separate out the organic layer, dry it for 2 hours with anhydrous magnesium sulfate. Then filter it. The filtrate is concentrated at reduced pressure to produce oily substance. The oily substance is purified by column chromatography (eluant: hexane/ethyl acetate 10:1). The target component is combined to produce a white solid LG-4-A: 6.24g (Yield: 80%).

### Step 2: Preparation of LG-4

Dissolve 4.0g LG-4-A (9mmol) in 50ml tetrahydrofura. Add 300mg LiAlH4 (7.9mmol) at the temperature cooled to below zero with ice-salt bath. Keep the reaction at 0□ for 30 minutes. Then add 0.3ml water, 0.9ml 15% NaOH solution, 0.3ml water in turn, and at this time a precipitate is generated. Filter out the precipitate. The filter cake is washed with tetrahydrofura for one time. The filtrate is combined and concentrated at reduced pressure to dry. The residue is purified by column chromatography to produce 1.65g LG-4 (Yield: 55.5%).

### Example 6: Preparation of HH-EPO-005

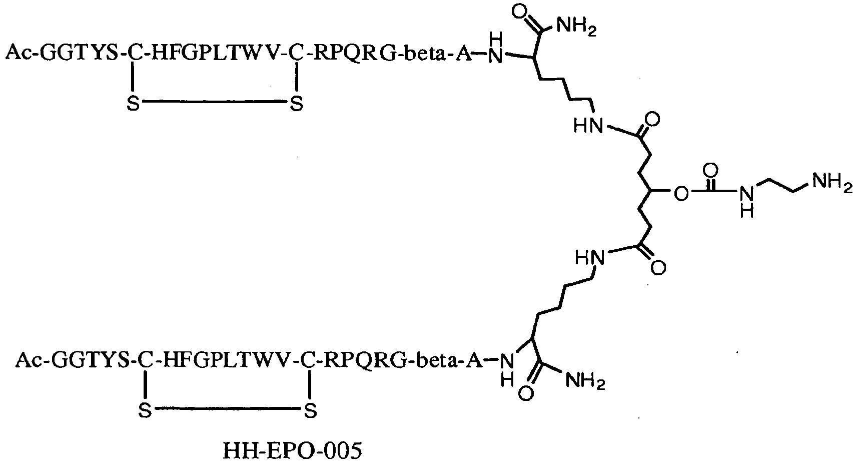

### Step 1: Preparation of SEQ ID NO: 5 Cyclic Peptides

Dissolve 9g EPO mimetic peptide derivative monomer peptide SEQ ID NO: 5 (synthesis in accordance with the method given in the Examples) in 3000ml 20% glacial acetic acid, and then slowly add 5% iodine methanol solution drop by drop until the yellow color is not disappearing. The reaction solution is directly subjected to preparative purification by reversed-phase chromatography using octadecyl silane bonded silica gel as column filler (Waters SymmetryShield™ RP18 3.5µm, 4.6*100mm), wherein the column temperature is 60 □ and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, 3.0g of SEQ ID NO: 5 cyclic peptide is prepared by freeze drying (Yield: 15.6%).

### Step 2: Preparation of HH-EPO-005

Dissolve SEQ ID NO: 5 cyclic peptide 3.0g (1.22mmol) in 150ml N,N-dimethyl formamide. Add triethylamine 147mg (1.46mmol), 368mg functional small molecules (LG-3) (0.61mmol). After stirring the reaction at room temperature for 6 hours, concentrate out part of the N,N-dimethylformamide. Add 200ml ether into the residue, place it in refrigerator for 2 hours and then centrifugate. A white solid is obtained by vacuum dry. Then dissolve this white solid in 50ml 20% trifluoroacetic acid/dichloromethane solution. After stirring at room temperature for 30 minutes, part of the solvent is concentrated out uner reduced pressure. Add 200ml ether into the residue, place it in refrigerator for 2 hours and then centrifugate. A white solid is obtained by vacuum dry. The white solid is subjected to preparative purification by reversed-phase chromatography, using octadecyl silane bonded silica gel as column filler (Waters SymmetryShield™ RP18 3.5µm, 4.6*100mm), wherein the column temperature is 60□ and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-005: 1.0g is prepared by freeze drying (Yield is about 33%).

### Example 7: Preparation of HH-EPO-006

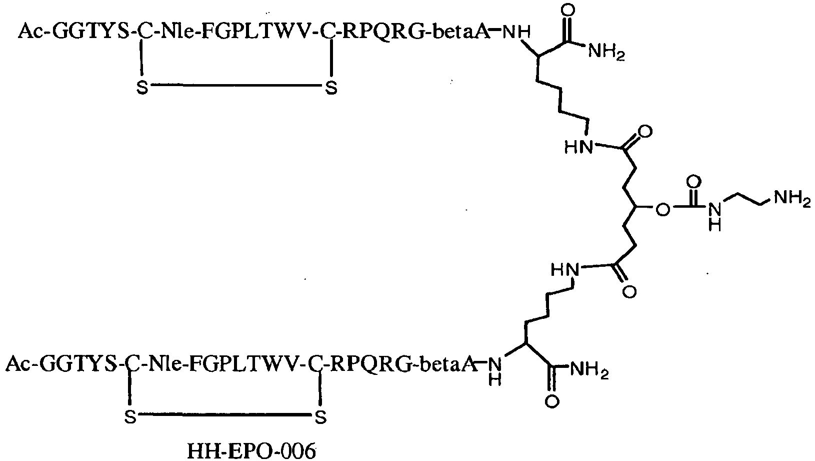

### Step1: Preparation of SEQ ID NO: 6 cyclic peptides

Dissolve 9g EPO mimetic peptide derivative monomer peptide SEQ ID NO: 6 (synthesis in accordance with the method given in the Examples) in 3000ml 20% glacial acetic acid, and then slowly add 5% iodine methanol solution drop by drop until the yellow color is not disappearing. The reaction solution is directly subjected to preparative purification by reversed-phase chromatography, using octadecyl silane bonded silica gel as column filler (Waters SymmetryShield™ RP18 3.5µm, 4.6*100mm), wherein the column temperature is 60 □ and the detection wavelength is 214nm; water (containing 0.05% trifluoroacetic acid) and acetonitrile (containing 0.05% trifluoroacetic acid) in different proportions are used as mobile phase. Combine and collect target fractions. After most of the acetonitrile are distilled off under reduced pressure, 3.0 g SEQ ID NO:6 cyclic peptide is prepared by freeze drying (Yield: 15.3%).

### Step2: Preparation of HH-EPO-006

Dissolve SEQ ID NO: 6 cyclic peptide 3.0g (1.22mmol) in 150ml N,N-dimethyl formamide. Add triethylamine 147mg (1.46mmol), 368mg functional small molecules (LG-3) (0.61mmol). After stirring the reaction at room temperature for 6 hours, concentrate out part of the DMF. Add 200ml ether into the residue, place it in refrigerator for 2 hours and then centrifugate. A white solid is obtained by vacuum dry. Then dissolve this white solid in 50ml 20% trifluoroacetic acid/dichloromethane solution. After stirring at room temperature for 30 minutes, part of the solvent is concentrated out uner reduced pressure. Add 200ml ether into the residue, place it in refrigerator for 2 hours and then centrifugate. A white solid is obtained by vacuum dry. The white solid is subjected to preparative purification by reversed-phase chromatography, using octadecyl silane bonded silica gel as column filler (Waters SymmetryShield^{™} RP18 3.5µm, 4.6*100mm), wherein the column temperature is 60°C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-006: 3.98g is prepared by freeze drying (Yield is about 32.7%).

### Example 8: Preparation of HH-EPO-007

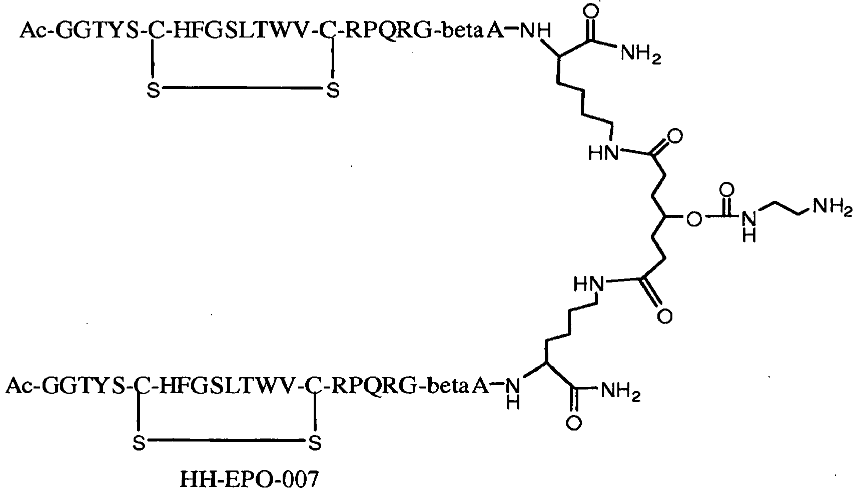

### Step1: Preparation of SEQ ID NO: 7 Cyclic Peptides

Dissolve 9g EPO mimetic peptide derivative monomer peptide SEQ ID NO: 7 (synthesis in accordance with the method given in the Examples) in 3000ml 20% glacial acetic acid, and then slowly add 5% iodine methanol solution drop by drop until the yellow color is not disappearing. The reaction solution is directly subjected to preparative purification by reversed-phase chromatography, using octadecyl silane bonded silica gel as column filler (Waters SymmetryShield™ RP18 3.5µm, 4.6*100mm), wherein the column temperature is 60 °C and the detection wavelength is 214nm; water (containing 0.05% trifluoroacetic acid) and acetonitrile (containing 0.05% trifluoroacetic acid) in different proportions are used as mobile phase. Combine and collect target fractions. After most of the acetonitrile are distilled off under reduced pressure, 3.15 g SEQ ID NO:7 cyclic peptide is prepared by freeze drying (Yield: 16.4%).

### Step2: Preparation of HH-EPO-007

Dissolve SEQ ID NO: 7 cyclic peptide 3.0g (1.22mmol) in 150ml N,N-dimethyl formamide. Add triethylamine 147mg (1.46mmol), 368mg functional small molecules (LG-3) (0.61mmol). After stirring the reaction at room temperature for 6 hours, concentrate out part of the N,N-dimethylformamide. Add 200ml ether into the residue, place it in refrigerator for 2 hours and then centrifugate. A white solid is obtained by vacuum dry. Then dissolve this white solid in 50ml 20% trifluoroacetic acid/dichloromethane solution. After stirring at room temperature for 30 minutes, part of the solvent is concentrated out uner reduced pressure. Add 200ml ether into the residue, place it in refrigerator for 2 hours and then centrifugate. A white solid is obtained by vacuum dry. The white solid is subjected to preparative purification by reversed-phase chromatography, using octadecyl silane bonded silica gel as column filler (Waters SymmetryShield^{™} RP18 3.5µm, 4.6*100mm), wherein the column temperature is 60°C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-007: 1.0g is prepared by freeze drying (Yield is about 33%).

### Example 9: Preparation of HH-EPO-008

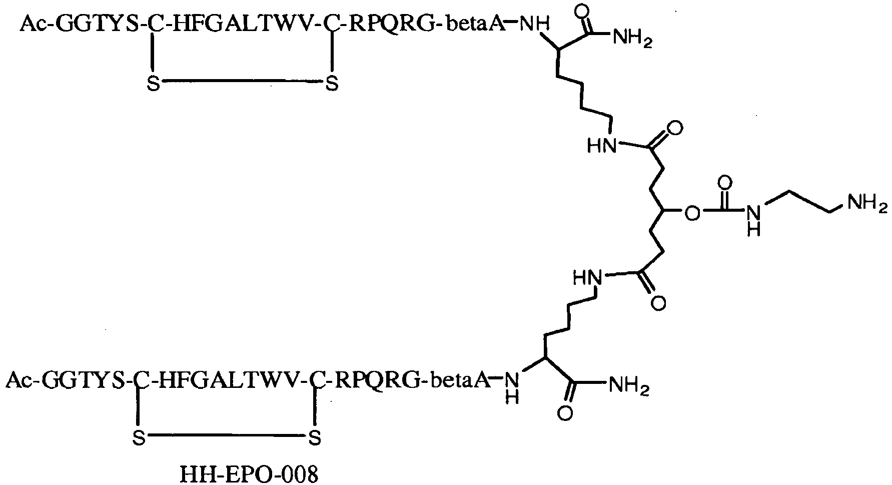

### Step1: Preparation of SEQ ID NO:8 Cyclic Peptides

Dissolve 27g EPO mimetic peptide derivative monomer peptide SEQ ID NO: 8 (synthesis in accordance with the method given in the Examples) in 3000ml 20% glacial acetic acid, and then slowly add 5% iodine methanol solution drop by drop until the yellow color is not disappearing. The reaction solution is directly subjected to preparative purification by reversed-phase chromatography, using octadecyl silane bonded silica gel as column filler (Waters SymmetryShield^{™} RP18 3.5µm, 4.6*100mm), wherein the column temperature is 60 °C and the detection wavelength is 214nm; water (containing 0.05% trifluoroacetic acid) and acetonitrile (containing 0.05% trifluoroacetic acid) in different proportions are used as mobile phase. Combine and collect target fractions. After most of the acetonitrile are distilled off under reduced pressure, 9.3 g SEQ ID NO:8 cyclic peptide is prepared by freeze drying (Yield: 15.7%).

### Step2: Preparation of HH-EPO-008

Dissolve SEQ ID NO: 8 cyclic peptide 3.0g (1.22mmol) in 150ml N,N-dimethyl formamide. Add triethylamine 147mg (1.46mmol), 368mg functional small molecules (LG-3) (0.61mmol). After stirring the reaction at room temperature for 6 hours, concentrate out part of the N,N-dimethylformamide. Add 200ml ether into the residue, place it in refrigerator for 2 hours and then centrifugate. A white solid is obtained by vacuum dry. Then dissolve this white solid in 50ml 20% trifluoroacetic acid/dichloromethane solution. After stirring at room temperature for 30 minutes, part of the solvent is concentrated out uner reduced pressure. Add 200ml ether into the residue, place it in refrigerator for 2 hours and then centrifugate. A white solid is obtained by vacuum dry. The white solid is subjected to preparative purification by reversed-phase chromatography, using octadecyl silane bonded silica gel as column filler (Waters SymmetryShield^{™} RP18 3.5µm, 4.6*100mm), wherein the column temperature is 60°C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-008: 1.12g is prepared by freeze drying (Yield is about 33%).

### Example 10: Preparation of HH-EPO-008A

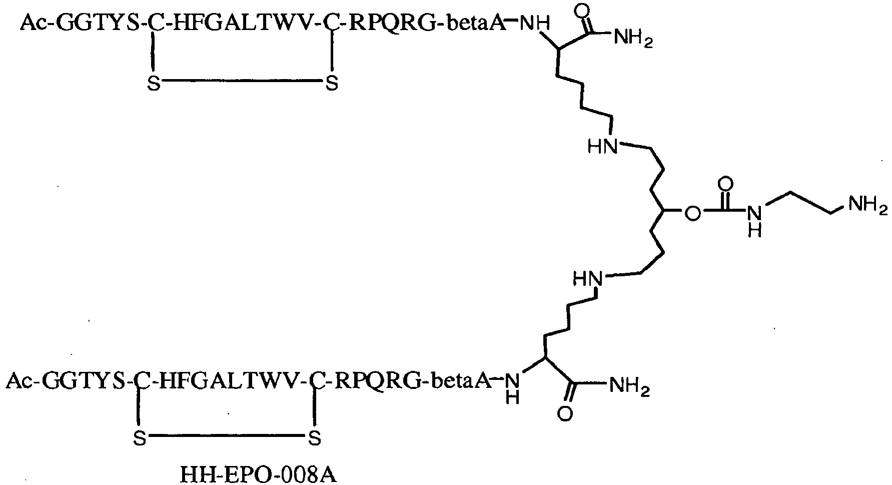

Dissolved SEQ ID NO: 8 cyclic peptide 3.0g (1.22mmol) in 150ml 20mmol acetic acid buffer (pH5.0), and then add 201mg functional small molecules (LG-4) (0.61mmol) and 10ml acetonitrile. After stirring the reaction at room temperature for 30 minutes, the reaction solution is subjected preparative purification by reversed-phase chromatography, using octadecyl silane bonded silica gel as column filler (Waters SymmetryShield^{™} RP18 3.5µm, 4.6*100mm), wherein the column temperature is 60 □ and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-008A: 0.75g is prepared by freeze drying (Yield: 25%).

### Example 11: Preparation of HH-EPO-008B

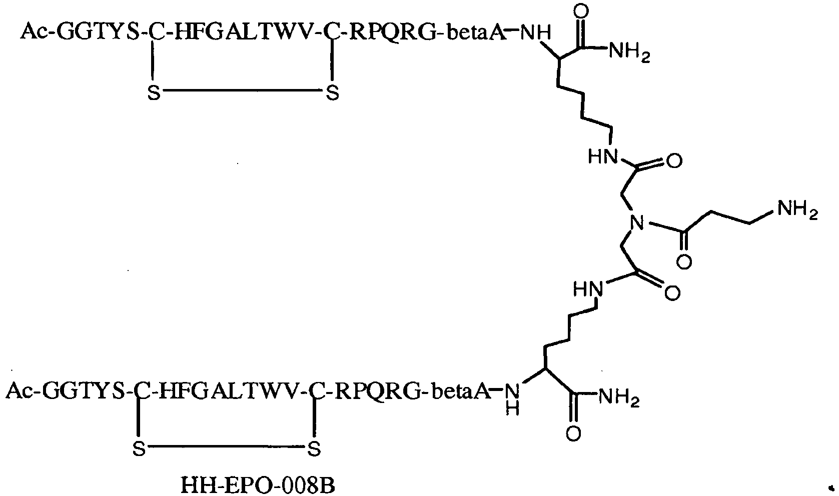

Dissolve SEQ ID NO: 8 cyclic peptide 3.0g (1.22mmol) in 150ml N,N-dimethyl formamide. Add triethylamine 147mg (1.46mmol), 322mg functional small molecules (LG-2) (0.61mmol). After stirring the reaction at room temperature for 6 hours, concentrate out part of the N,N-dimethylformamide. Add 200ml ether into the residue, place it in refrigerator for 2 hours and then centrifugate. A white solid is obtained by vacuum dry. Then dissolve this white solid in 50ml 20% trifluoroacetic acid/dichloromethane solution. After stirring at room temperature for 30 minutes, part of the solvent is concentrated out uner reduced pressure. Add 200ml ether into the residue, place it in refrigerator for 2 hours and then centrifugate. A white solid is obtained by vacuum dry. The white solid is subjected to preparative purification by reversed-phase chromatography, using octadecyl silane bonded silica gel as column filler (Waters SymmetryShield™ RP18 3.5µm, 4.6*100mm), wherein the column temperature is 60°C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-008: 1.3g is prepared by freeze drying (Yield is about 43%).

### Example 12: Preparation of HH-EPO-008C

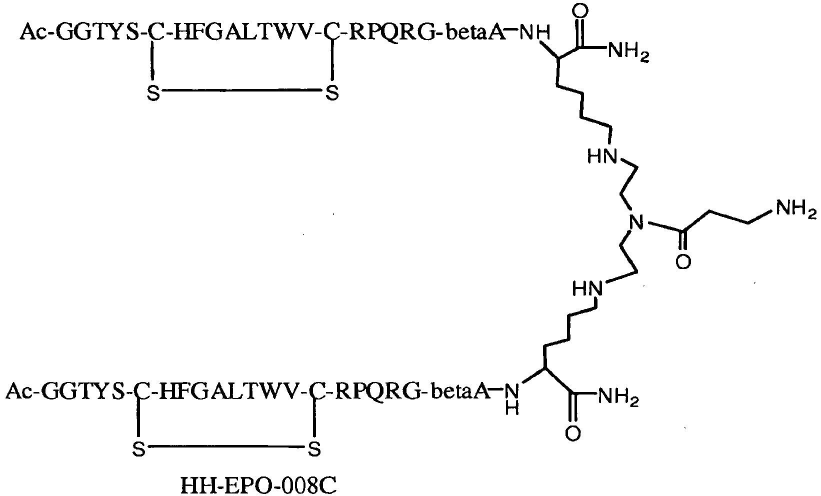

Dissolved SEQ ID NO: 8 cyclic peptide 3.0g (1.22mmol) in 150ml 20mmol acetic acid buffer (pH5.0), and then add 165mg functional small molecules (LG-1) (0.61mmol) and 10ml acetonitrile. After stirring the reaction at room temperature for 30 minutes, the reaction solution is subjected preparative purification by reversed-phase chromatography, using octadecyl silane bonded silica gel as column filler (Waters SymmetryShield™ RP18 3.5µm, 4.6*100mm), wherein the column temperature is 60 °C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-008C: 0.8g is prepared by freeze drying (Yield: 27%).

### Example 13: Preparation of HH-EPO-018

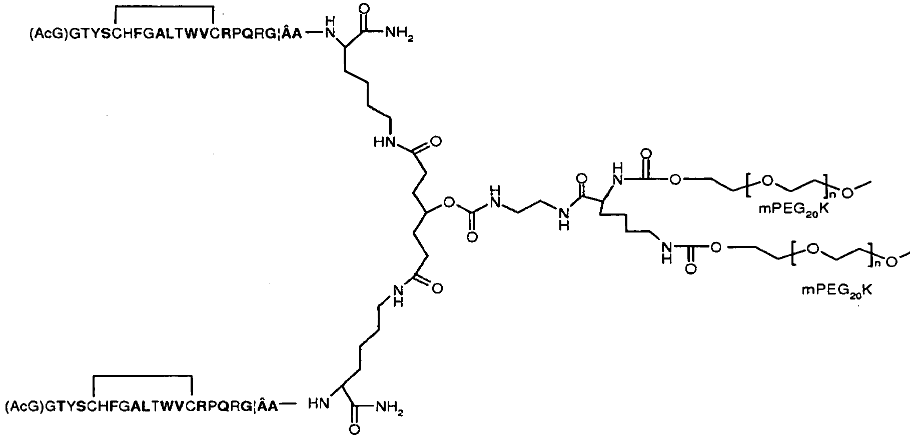

Dissolve 0.5g HH-EPO-008(0.98mmol) in 100ml N, N-dimethyl formamide, add triethylamine 39.6mg (0.196mmol), 3.8g mPEG₂-OSU(40K)(0.96mmol). Stir the reaction at room temperature for 6 hours. Put the reaction solution directly into 600ml cold ether. The solid is precipitated. After placing in refrigerator 2 hours, centrifugate it and crude HH-EPO-018 is obtained by vacuum dry. Crude HH-EPO-018 is purified by reversed-phase chromatography, using octadecyl silane bonded silica gel as column filler (Waters SymmetryShield™ RP18 3.5µm, 4.6*100mm), wherein the column temperature is 60 □ and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-018: 1.8g is prepared by freeze drying (Yield is about 47%).

### Example 14: Preparation of HH-EPO-018A

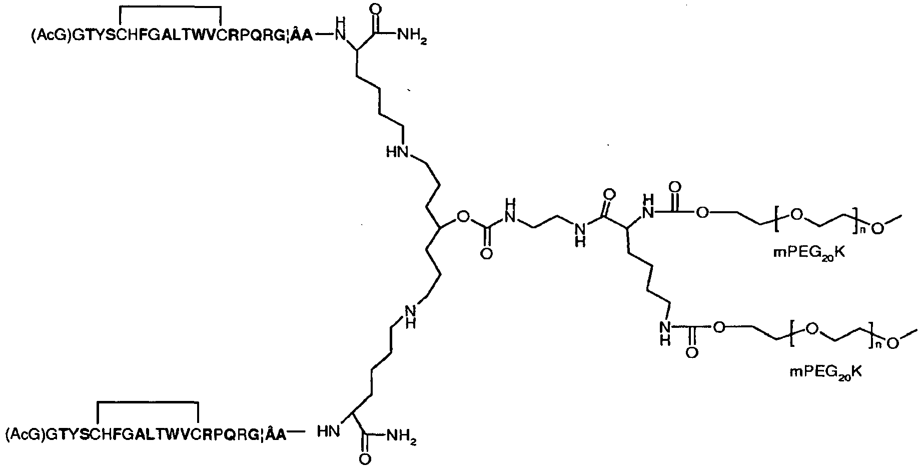

Dissolve 0.5g HH-EPO-008(0.98mmol) in 100ml N, N-dimethyl formamide, add triethylamine 39.6mg (0.196mmol), 3.8g mPEG₂-OSU(40K)(0.96mmol). Stir the reaction at room temperature for 6 hours. Put the reaction solution directly into 600ml cold ether. The solid is precipitated. After placing in refrigerator 2 hours, centrifugate it and crude HH-EPO-018 is obtained by vacuum dry. Crude HH-EPO-018 is purified by reversed-phase chromatography, using octadecyl silane bonded silica gel as column filler (Waters SymmetryShield^{™} RP18 3.5µm, 4.6*100mm), wherein the column temperature is 60 °C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-018A: 1.5g is prepared by freeze drying (Yield is about 39%).

### Example 15: Preparation of HH-EPO-018B

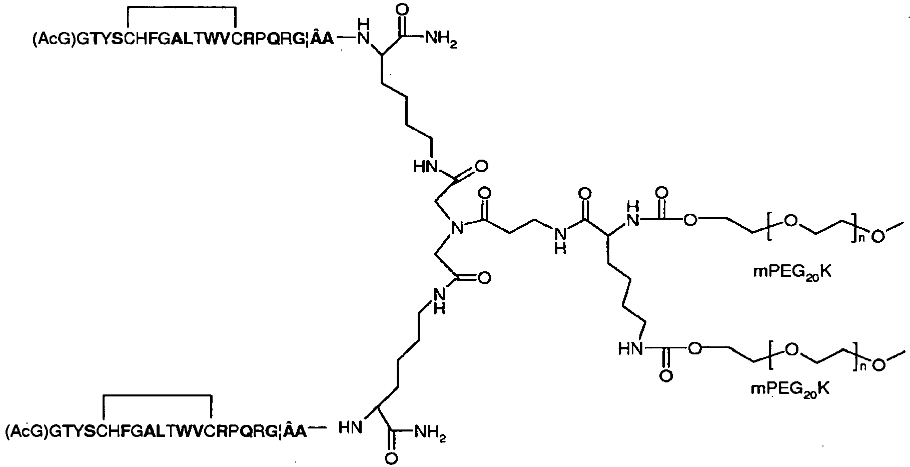

Dissolve 0.5g HH-EPO-008(0.98mmol) in 100ml N,N-dimethyl formamide, add triethylamine 39.6mg (0.196mmol), 3.8g mPEG₂-OSU(40K)(0.96mmol). Stir the reaction at room temperature for 6 hours. Put the reaction solution directly into 600ml cold ether. The solid is precipitated. After placing in refrigerator 2 hours, centrifugate it and crude HH-EPO-018 is obtained by vacuum dry. Crude HH-EPO-018 is purified by reversed-phase chromatography, using octadecyl silane bonded silica gel as column filler (Waters SymmetryShield^{™} RP18 3.5µm, 4.6*100mm), wherein the column temperature is 60 °C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-018: 1.7g is prepared by freeze drying (Yield is about 45%).

### Example 16: Preparation of HH-EPO-018C

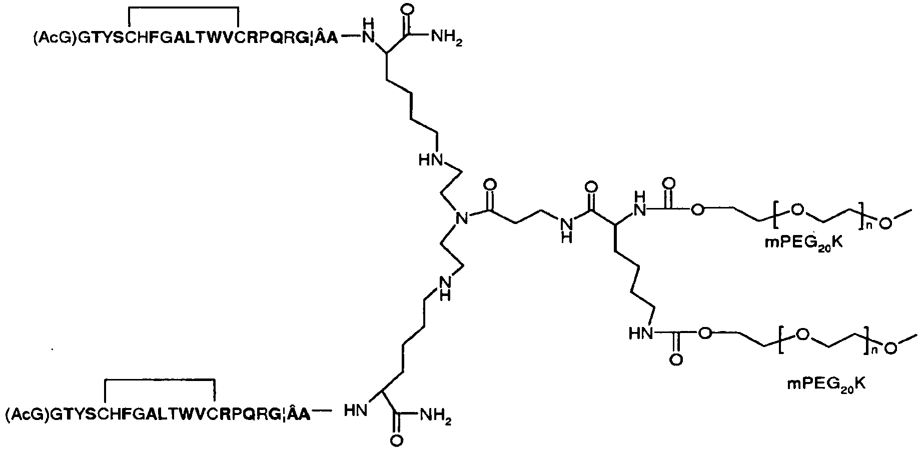

Dissolve 0.5g HH-EPO-008(0.98mmol) in 100ml N, N-dimethyl formamide, add triethylamine 39.6mg (0.196mmol), 3.8g mPEG₂-OSU(40K)(0.96mmol). Stir the reaction at room temperature for 6 hours. Put the reaction solution directly into 600ml cold ether. The solid is precipitated. After placing in refrigerator 2 hours, centrifugate it and crude HH-EPO-018 is obtained by vacuum dry. Crude HH-EPO-018 is purified by reversed-phase chromatography, using octadecyl silane bonded silica gel as column filler (Waters SymmetryShield^{™} RP18 3.5µm, 4.6*100mm), wherein the column temperature is 60 °C and the detection wavelength is 214nm; water (containing 0.05% trifluoroacetic acid) and acetonitrile (containing 0.05% trifluoroacetic acid) in different proportions are used as mobile phase. Combine and collect the target fractions. After most of the acetonitrile are distilled off under reduced pressure, HH-EPO-018: 1.4g is prepared by freeze drying (Yield is about 37%).

### Example 17: Effects of EPO mimetic peptide derivatives on mice

### Purpose of this experiment

To evaluate and compare the effects of EPO mimetic peptide derivatives and the EPO protein on erythropoiesis of mice.

### Materials and methods:

EPO mimetic peptide derivatives including HH-EPO-001, HH-EPO-002, HH-EPO-003, HH-EPO-004, HH-EPO-005, HH-EPO-006, HH-EPO-007, HH-EPO-008, HH-EPO-015, HH-EPO-016, HH-EPO-017 and HH-EPO-018 are provided by Jiangsu Hansoh Pharmaceutical co.,LTD. EPO is purchased from Shenyang Sansheng Pharmaceutical Co., Ltd. Kunming mice are purchased from the Chinese Academy of Sciences Shanghai Experimental Animal Center, weighing 25 ∼ 30g, ♀. The number of animals in each group is 10.

Mice were injected subcutaneously with EPO mimetic peptide derivatives and EPO protein for three consecutive days. Then kill the mice, take whole blood to carry out peripheral blood cells and reticulocyte counts Blood cell count are performed using automatic blood cell counter counts.

### Results and discussion

According to the current dosage regimen, both EPO mimetic peptide derivatives and the EPO protein can significantly promote the mouse peripheral blood reticulocyte count to increase, indicating that they stimulate erythropoiesis (See Table 1). EPO mimetic peptide derivatives and the EPO protein have no significant influence on mature red blood cells, blood cell hematocrit, hemoglobin content (See Table 2), and also have no significant influence on the peripheral white blood cell count (See Table 3).

**Table 1 Effects of EPO mimetic peptide derivatives on mouse reticulocyte erythropoiesis.**

| Group | mouse (number) | Dose and programs | reticulocyte count (×10⁹/L,x±SD) |
|---|---|---|---|
| control | 10 | 0.1%BSA in NS | 136.9±5.6 |
| HH-EPO-005 | 10 | 4.5mg/kg,sc,d1-3 | 947.2±14.7 |
| HH-EPO-006 | 10 | 4.5mg/kg,sc,d1-3 | 515.0±22.7 |
| HH-EPO-007 | 10 | 4.5mg/kg,sc,d1-3 | 553.5±26.6 |
| HH-EPO-008 | 10 | 4.5mg/kg,sc,d1-3 | 908.1±21.7 |
| HH-EPO-015 | 10 | 4.5mg/kg,sc,d1-3 | 1146.9±176.6 |
| HH-EPO-016 | 10 | 4.5mg/kg,sc,d1-3 | 1796.4±304.4 |
| HH-EPO-017 | 10 | 4.5mg/kg,sc,d1-3 | 1208.9±178.5 |
| HH-EPO-018 | 10 | 4.5mg/kg,sc,d1-3 | 2000.6±272.0 |
| HH-EPO-018A | 10 | 4.5mg/kg,sc,d1-3 | 1889.3±252.0 |
| HH-EPO-018B | 10 | 4.5mg/kg,sc,d1-3 | 1969.7±312.0 |
| HH-EPO-018C | 10 | 4.5mg/kg,sc,d1-3 | 1879.3±162.0 |
| EPO | 10 | 5µg/kg,sc,d1-3 | 483.9±146.5 |

**Table 2 Effects of EPO mimetic peptide derivatives on mouse erythropoiesis, blood cell hematocrit, hemoglobin content.**

| group | mouse(number) | Dose and programs | Red blood cell count (×10⁶/µL, x±SD) | blood cell hematocrit (%) | hemoglobin (%) |
|---|---|---|---|---|---|
| control | 10 | 0.1 %BSA in NS | 9.6±0.5 | 48.2±3.0 | 14.8±0.7 |
| HH-EPO-005 | 10 | 4.5mg/kg,sc,d1-3 | 10.1±0.6 | 54.4±3.2 | 16.3±0.9 |
| HH-EPO-006 | 10 | 4.5mg/kg,sc,d1-3 | 9.6±0.5 | 50.5±2.8 | 15.3±0.9 |
| HH-EPO-007 | 10 | 4.5mg/kg,sc,d1-3 | 9.1±3.1 | 49.4±17.1 | 14.8±4.8 |
| HH-EPO-008 | 10 | 4.5mg/kg,sc,d1-3 | 9.6±0.2 | 54.0±1.7 | 16.1±0.5 |
| HH-EPO-015 | 10 | 4.5mg/kg,sc,d1-3 | 10.0±0.40 | 54.57±2.50 | 15.01±0.57 |
| HH-EPO-016 | 10 | 4.5mg/kg,sc,d1-3 | 9.88±0.42 | 56.50±2.95 | 13.24±4.2 |
| HH-EPO-017 | 10 | 4.5mg/kg,sc,d1-3 | 9.70±0.30 | 55.84±2.33 | 14.93±0.55 |
| HH-EPO-018 | 10 | 4.5mg/kg,sc,d1-3 | 9.69±0.33 | 56.97±3.13 | 13.22±2.66 |
| HH-EPO-018A | 10 | 4.5mg/kg,sc,d1-3 | 9.44±0.65 | 54.47±2.61 | 14.35±1.35 |
| HH-EPO-018B | 10 | 4.5mg/kg,sc,d1-3 | 9.77±0.51 | 55.71±3.31 | 13.72±2.35 |
| HH-EPO-018C | 10 | 4.5mg/kg,sc,d1-3 | 9.59±0.53 | 54.98±2.83 | 13.86±2.47 |
| EPO | 10 | 5µg/kg,sc,d1-3 | 9.0±0.6 | 46.2±2.7 | 14.3±0.7 |

**Table 3 Effects of EPO mimetic peptide derivatives on mouse platelets, white blood cell generation**

| group | mouse(number) | Dose and programs | platelet(×10³/µL) | white blood cell(×10³/µL) |
|---|---|---|---|---|
| control | 10 | 0.1%BSA in NS | 1078.0±151.2 | 5.1±1.5 |
| HH-EPO-005 | 10 | 4.5mg/kg,sc,d1-3 | 1957.8±349.5 | 4.2±1.2 |
| HH-EPO-006 | 10 | 4.5mg/kg,sc,d1-3 | 1087.8±118.5 | 4.1±1.2 |
| HH-EPO-007 | 10 | 4.5mg/kg,sc,d1-3 | 2082.1±863.9 | 3.6±0.8 |
| HH-EPO-008 | 10 | 4.5mg/kg,sc,d1-3 | 1685.5±351.3 | 2.9±0.5 |
| HH-EPO-015 | 10 | 4.5mg/kg,sc,d1-3 | 1106.6±170.03 | 4.32±1.29 |
| HH-EPO-016 | 10 | 4.5mg/kg,sc,d1-3 | 1275.88±239.90 | 5.06±1.41 |
| HH-EPO-017 | 10 | 4.5mg/kg,sc,d1-3 | 1109.60±130.73 | 4.25±1.65 |
| HH-EPO-018 | 10 | 4.5mg/kg,sc,d1-3 | 1317.50±461.06 | 4.11±1.31 |
| HH-EPO-018A | 10 | 4.5mg/kg,sc,d1-3 | 1432.50±453.05 | 4.23±1.23 |
| HH-EPO-018B | 10 | 4.5mg/kg,sc,d1-3 | 1337.70±363.06 | 4.07±1.23 |
| HH-EPO-018C | 10 | 4.5mg/kg,sc,d1-3 | 1355.50±331.07 | 4.21±1.34 |
| EPO | 10 | 5µg/kg,sc,d1-3 | 1306.8±170. | 4.0±0.9 |

### Example 18: Effects of EPO mimetic peptide derivatives on Macaques

### Purpose of this experiment:

To evaluate the effects of EPO mimetic peptide derivatives on erythropoiesis of the Macaques.

### Materials and methods::

EPO memitic peptide derivatives HH-EPO-018 is provided by Jiangsu Hansoh Pharmaceutical co.,LTD. EPO is purchased from Shenyang Sansheng Pharmaceutical Co., Ltd. Dilute it in saline containing 0.1% BSA before it is used.

Macaques, weighing 5.5∼8.5kg, male or female, are purchased from Suzhou Xishan Zhongke Laboratory Animal Center. Macaques are grouped according to the basis of hemoglobin with each group three macaques. HH-EPO-018 1.35mg/kg is intravenously injected once; EPO 240µ/kg, three times/week, continuous administration for five weeks. Mesure hematological indexes 1∼2 times per week.

### Results and discussion

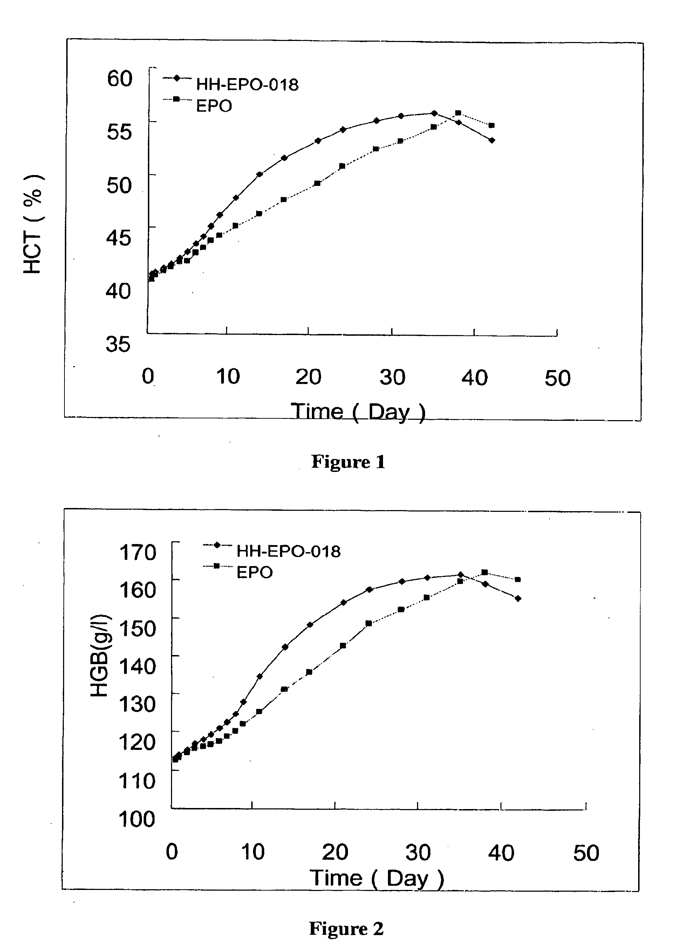

Single intravenous injection of HH-EPO-018 on macaques leads to an increase of peripheral blood hemoglobin content, an increase of blood cell hematocrit, indicating that HH-EPO-018 stimulate erythropoiesis. The stimulation peaked at 35 days after administration, and then decreased slowly. The stimulatory effect of hemoglobin is about 33%. As positive control, EPO also give the same increase in macaque peripheral blood hemoglobin content and blood cell hematocrit, and its effect declines slowly after stopping administering the medicine. According to the current dosage regimen, the stimulation of HH-EPO-018 and EPO on macaque hemoglobin generation is considerable (see figures 1, 2).

### Example 19: To evaluate and compare the effects of EPO mimetic peptide derivatives HH-EPO-015, HH-EPO-018, HH-EPO-018B and positive control AF37702 on the mice.

### Materials and methods:

HH-EPO-015, HH-EPO-018, HH-EPO-018B and AF37702 are provided by Jiangsu Hansoh Pharmaceutical co.,LTD, wherin AF37702 is also EPO mimetic peptide derivative, produced by Affymax (brand name: Hematide). Prepare the sample in saline containing 0.1% BSA before it is used. Kunming mice are purchased from the Chinese Academy of Sciences Shanghai Experimental Animal Center, weighing 25 ∼ 30g, ♀. The number of animals in each group is 10. After adaptation, animals are subcutaneously injected with HH-EPO-015, HH-EPO-018, HH-EPO-018B, AF37702. Kill the mice on the sixth day after the first dose, take whole blood to carry out peripheral blood cells and reticulocyte count. Blood cell count are performed using ADVIA automatic blood cell counter counts.

### Results and discussion

A single subcutaneous injection of HH-EPO-015, HH-EPO-018, HH-EPO-018B, AF37702 all significantly elevate the mouse peripheral blood reticulocyte percentage and counts; wherein the effects of HH-EPO-018B is relatively strong; the effects of HH-EPO-018, AF37702 followed; the effects of HH-EPO-015 is the weakest; the effects of HH-EPO-018 and AF37702 is roughly equal (see Table 4). HH-EPO-015, HH-EPO-018, HH-EPO-018B and AF37702 elevate the mouse peripheral blood cell hematocrit, hemoglobin content. Their effects are roughly equal, but they all have no significant influence on the peripheral red blood cell count (See Table 5).

**Table4: Effcts of HH-EPO-015, HH-EPO-018, HH-EPO-018B and AF37702 on the mouse peripheral blood reticulocyte erythropoiesis**

| group | mouse(number) | Dose and programs | reticulocyte erythropoiesis (x±SD) | reticulocyte erythropoiesis counting (×10⁹/L,x±SD) |
|---|---|---|---|---|
| control | 10 | 0.1 %BSA in NS | 2.8±1.0 | 195.0±73.1 |
| HH-EPO-015 | 10 | 2.5mg/kg,sc,d1 | 6.9±2.1** | 511.9±191.7** |
| HH-EPO-015 | 10 | 5.0 mg/kg,sc,d1 | 8.9±2.4** | 558.9±230.5** |
| HH-EPO-018 | 10 | 2.5mg/kg,sc,d1 | 16.2±3.5** | 1137.3±240.2** |
| HH-EPO-018 | 10 | 5.0 mg/kg,sc,d1 | 16.0±3.2** | 1113.2±210.7** |
| HH-EPO-018B | 10 | 2.5mg/kg,sc,d1 | **19.0±8.9**** | 1336.5±629.0** |
| HH-EPO-018B | 10 | 5.0 mg/kg,sc,d1 | **20.0±5.3**** | 1440.3±416.5** |
| AF37702 | 10 | 2.5mg/kg,sc,d1 | 13.5±4.1** | 865.2±291.4** |
| AF37702 | 10 | 5.0mg/kg,sc,d1 | 17.2±5.3** | 1202.8±355.4** |

| | | | | |
|---|---|---|---|---|
| **P<0.01 vs control | | | | |

**Table 5: Effects of HH-EPO-015,HH-EPO-018,HH-EPO-018B and AF37702 on mouse peripheral erythropoiesis, blood cell hematocrit, hemoglobin content.**

| group | mouse (numb er) | Dose and programs | Red blood cell count (×10⁶/uL,x±SD) | blood cell hematocrit (×10⁹/L,x±SD) | hemoglob in (g/dL) |
|---|---|---|---|---|---|
| control | 10 | 0.1%BSA in NS | 6.9±0.5 | 38.6±2.8 | 12.5±0.9 |
| HH-EPO-015 | 10 | 2.5mg/kg,sc,d1 | 7.5±0.3 | 42.7±1.8* | 14.3±0.6** |
| HH-EPO-015 | 10 | 5.0 mg/kg,sc,d1 | 6.3±1.7 | 36.5±9.9 | 13.1±4.5 |
| HH-EPO-018 | 10 | 2.5mg/kg,sc,d1 | 7.0±0.3 | 40.6±1.6 | 13.3±2.2 |
| HH-EPO-018 | 10 | 5.0 mg/kg,sc,d1 | 7.0±0.3 | 41.5±1.2* | 14.2±0.8** |
| HH-EPO-018B | 10 | 2.5mg/kg,sc,d1 | 6.6±1.4 | 39.0±7.7 | 14.2±0.6** |
| HH-EPO-018B | 10 | 5.0 mg/kg,sc,d1 | 7.0±0.4 | 41.6±2.6* | 14.4±0.9** |
| AF37702 | 10 | 2.5mg/kg,sc,d1 | 7.1±0.4 | 41.9±3.1* | 14.1±1.1** |
| AF37702 | 10 | 5.0mg/kg,sc,d1 | 7.2±0.3 | 42.5±3.4* | 14.0±0.8** |

| | | | | | |
|---|---|---|---|---|---|
| *P<0.05, **P<0.01 vs control | | | | | |

## Claims

1. An erythropoietin (EPO) mimetic peptide derivative of general formula (I) and its pharmaceutical acceptable salts,
R₁-R₂-(CH₂)ₙ₁-R₃-(CH₂)ₙ₂-R₄-R₅ (I)
wherein R₁,R₅ are selected from cyclic peptides having the following sequences: wherein n₁,n₂ are integers independently selected from 0 to 10;
wherein R₂, R₄ are selected from -CO or -CH₂;
wherein R₃ is selected from NCO(CH₂)ₙ₄NHR₆, CHOCONH(CH₂)ₙ₅NHR₆, or CHSCON(CH₂)ₙ₅NHR₆;
wherein n₄ is an integer selected from 2 to 10,
wherein n₅ is an integer selected from 2 to 10,
and wherein R₆ is selected from H or methoxy polyethylene glycol derivatives.

2. The EPO mimetic peptide derivative of claim 1 and its pharmaceutical acceptable salts, wherein the amino acid sequence of R₁, R₅ might be consistent or inconsistent.

3. The EPO mimetic peptide derivative of any one of claims 1 to 2 and its pharmaceutical acceptable salts, wherein N-terminal of R₁, R₅ are acetylized.

4. The EPO mimetic peptide derivative of any one of claims 1 to 3 and its pharmaceutical acceptable salts, wherein R₁, R₅ are cyclic peptide cyclized by disulfide bonds.

5. The EPO mimetic peptide derivative of any one of claims 1 to 4 and its pharmaceutical acceptable salts, wherein R₁, R₅ are separately selected from cyclic peptides having the following sequences:

6. The EPO mimetic peptide derivative of any one of claims 1 to 5 and its pharmaceutical acceptable salts, wherein said EPO mimetic peptide is selected from the following peptide, wherein
n₁, n₂ are 2, R₂, R₄ are -CO, R₃ is CHOCONH(CH₂)ₙ₅NHR₆, n₅ is 2;
n₁, n₂ are 1, R₂, R₄ are -CO, R₃ is NCO(CH₂)ₙ₄NHR₆, n₄ is 2:
n₁, n₂ are 2, R₂, R₄ are -CH₂, R₃ is CHOCONH(CH₂)ₙ₅NHR₆, n₅ is 2; or
n₁, n₂ are 1, R₂, R₄ are -CH₂, R₃ is NCO(CH₂)ₙ₄NHR₆,n₄ is 2.

7. The EPO mimetic peptide derivative of any one of claims 1 to 6 and its pharmaceutical acceptable salts, **characterized in that** R₆ is H.

8. The EPO mimetic peptide derivative of any one of claims 1 to 7 and its pharmaceutical acceptable salts, wherein R₆ is methoxy polyethylene glycol derivatives, and wherein the molecular weight of methoxy polyethylene glycol derivatives is preferably selected from 5,000 to 100,000 Dalton.

9. The EPO mimetic peptide derivative of claim 8 and its pharmaceutical acceptable salts, wherein the structure of methoxy polyethylene glycol derivatives is selected from the branched or linear type, preferably the methoxy polyethylene glycol derivatives is linear structure have molecular weight of 20,000 daltons, or methoxy polyethylene glycol derivatives is linear structure and have molecular weight of 40,000 daltons.

10. The EPO mimetic peptide derivative of any one of claims 1 to 9 and its pharmaceutical acceptable salts, wherein
n₁, n₂ are 2, R₁, R₅ are selected from SEQ ID NO: 5 to SEQ ID NO: 8, R₂, R₄ are selected from-CO,-CH₂, R₃ is CHOCONH (CH₂) n₅NHR₆, wherein n₅ is an integer selected from 2 to 10; R₆ is a methoxy polyethylene glycol derivatives with linear structure and molecular weight of 20,000 daltons;
n₁, n₂ are 1, R₁, R₅ are selected from SEQ ID NO: 5 to SEQ ID NO: 8, R₂, R₄ are selected from-CO,-CH₂, R₃ is selected from NCO(CH₂)ₙ₄NHR₆, wherein n₄ is an integer selected from 2 to 10; R₆ is a methoxy polyethylene glycol derivatives with linear structure and molecular weight of 20,000 daltons;
n₁, n₂ are 2, R₁, R₅ are selected from SEQ ID NO: 5 to SEQ ID NO: 8, R₂, R₄ are selected from-CO,-CH₂, R₃ is CHOCONH(CH₂)ₙ₅NHR₆, wherein n₅ is an integer selected from 2 to 10; R₆ is a methoxy polyethylene glycol derivatives with linear structure and molecular weight of 40,000 daltons; or
n₁, n₂ are 1, R₁, R₅ are selected from SEQ ID NO: 5 to SEQ ID NO: 8, R₂, R₄ are selected from-CO,-CH₂, R₃ is NCO(CH₂)ₙ₄NHR₆, wherein n₄ is an integer selected from 2 to 10; R₆ is a methoxy polyethylene glycol derivatives with linear structure and molecular weight of 40,000 daltons.

11. The EPO mimetic peptide derivative of any one of claims 1 to 10 and its pharmaceutical acceptable salts, wherein said EPO mimetic peptide derivative and its pharmaceutical acceptable salts is

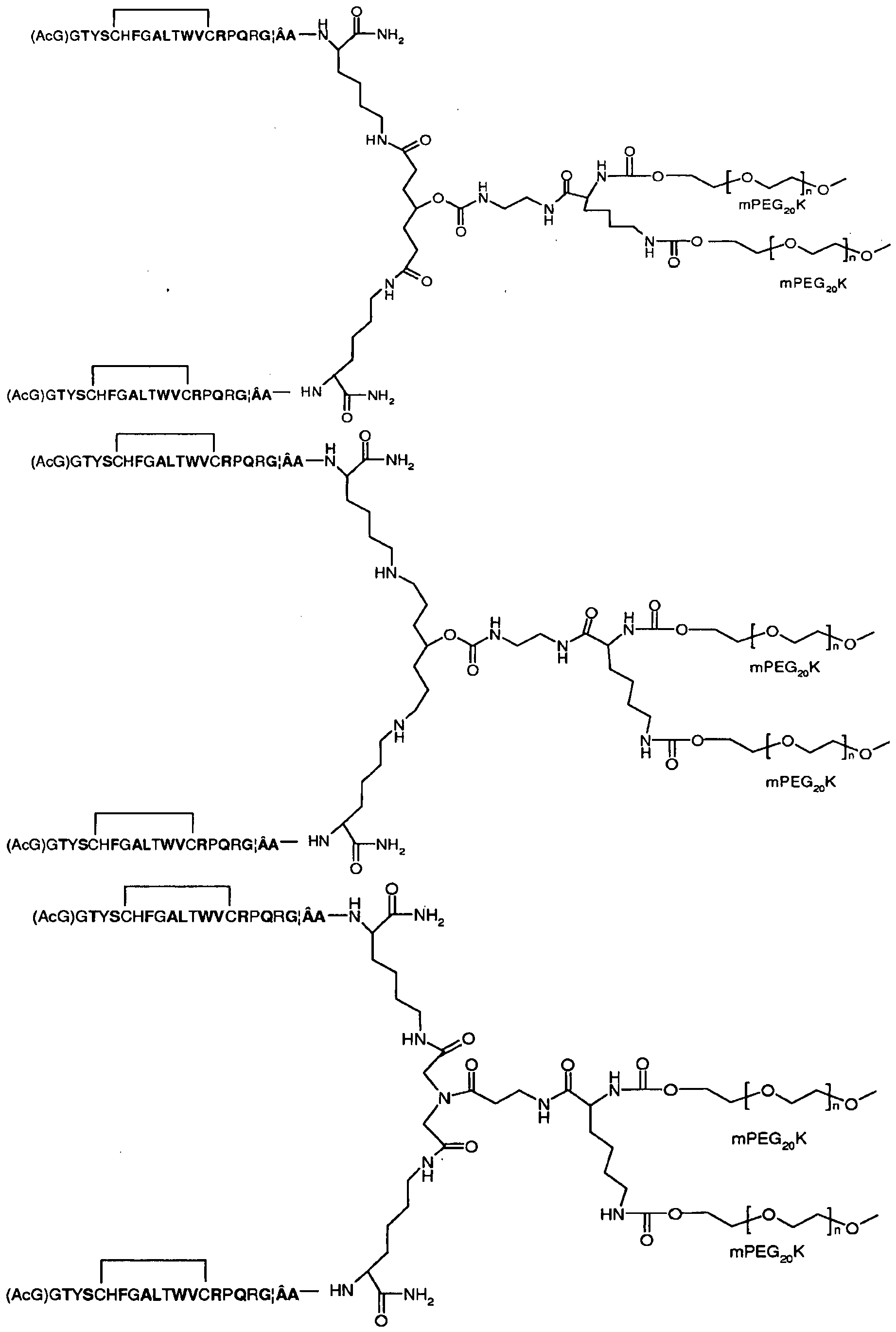

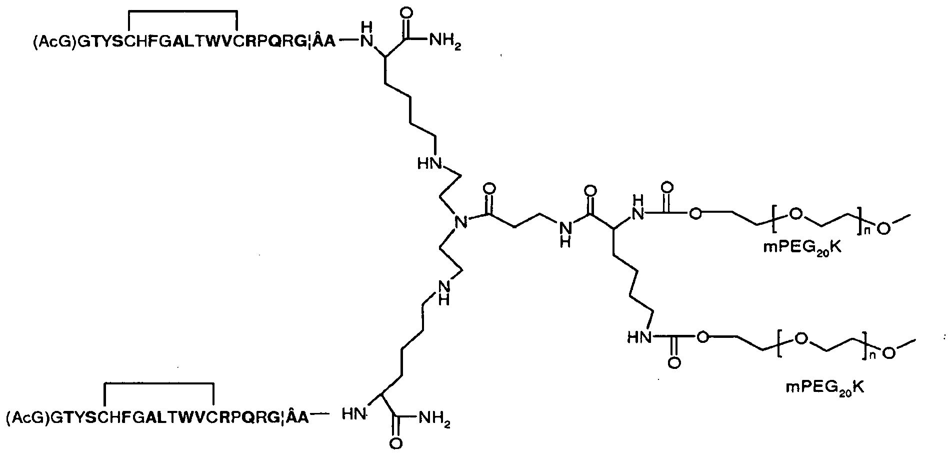

12. A method for preparation of EPO mimetic peptide derivative of any one of claims 1 to 11 and its pharmaceutical acceptable salts thereof, comprising:
(1) preparing R₁H, R₅H, wherein R₁, R₅ are selected from cyclic peptides having the sequences of SEQ ID NOs: 5-8, SEQ ID NOs: 12-20, SEQ ID NOs: 23-26, SEQ ID NOs 29-30;
(2) preparing functional small molecule of general formula (II)
R₇-CO-(CH₂)ₙ₁-Z₂-(CH₂)ₙ₂-CO-R₈ (II)
wherein n₁, n₂ are integers independently selected from 0 to 10;
R₇, R₈ are selected from OH or H;
Z₂ is selected from NCO(CH₂)ₙ₇NHR₉, CHOCONH(CH₂)ₙ₈NHR₉, or CHSCON(CH₂)ₙ₈NHR₉, wherein n₇ is an integer selected from 2 to 10, n₈ is an integer selected from 2 to 10, R₉ is selected from Boc or Cbz;
(3) reacting R₁,R₅ with functional small molecule of formula (II) through amidation or reductive amination to prepare the compound of formula (III),
R₁-R₂-(CH₂)ₙ₁-Z₂-(CH₂)ₙ₂-R₄-R₅ (III),
wherein R₂,R₄ are independently selected from -CO or -CH₂;
(4) after Boc or Cbz is removed, carrying out amidation reaction with active methoxy polyethylene glycol.

13. The method for the preparation of claim 12, **characterized in that** in said general formula (II)
n₁, n₂ are 1 or 2, R₇, R₈ are OH, Z₂ is selected from NCO(CH₂)ₙ₇NHR₉ or CHOCONH(CH₂)ₙ₈NHR₉, n₇ is an integer selected from 2 to 10, n₈ is an integer selected from 2 to 10, R₉ is Boc; or
n₁, n₂ are 1 or 2, R₇, R₈ are H, Z₂ is selected from NCO(CH₂)ₙ₇NHR₉ or CHOCONH(CH₂)ₙ₈NHR₉, n₇ is an integer selected from 2 to 10, n₈ is an integer selected from 2 to 10, R₉ is Boc.

14. A pharmaceutical composition comprising:
(1) a therapeutically effective amount of the EPO mimetic peptide derivative of any one of claims 1 to 11 or pharmaceutical acceptable salts thereof, and
(2) pharmaceutically acceptable carriers.

15. Use of the EPO mimetic peptide derivative of any one of claims 1 to 11 or pharmaceutical acceptable salts thereof or a pharmaceutical composition of claim 14, in the preparation of a medicament for the treatment of disorders **characterized by** a low level of EPO or insufficient or defective red blood cell group.

16. The use of claim 15, wherein said disorders **characterized by** a low level of EPO or insufficient or defective red blood cell group are advanced renal failure or dialysis; AIDS-related anemia, autoimmune diseases, or malignant tumor; cystic fibrosis; early premature anemia; chronic inflammatory disease-related anemia; spinal cord injury; acute blood loss; aging and cancer with abnormal red blood cells produced.

## Patentansprüche

1. Erythropoietin-(EPO)-mimetisches Peptidderivat der allgemeinen Formel (I) und seine pharmazeutisch akzeptablen Salze,
R₁-R₂-(CH₂)ₙ₁-R₃-(CH₂)n₂-R₄-R₅ (I)
wobei R₁ und R₅ aus zyklischen Peptiden mit den folgenden Sequenzen ausgewählt sind: wobei n₁ und n₂ ganze Zahlen sind, die unabhängig aus 0 bis 10 ausgewählt sind,
wobei R₂ und R₄ aus -CO oder -CH₂ ausgewählt sind,
wobei R₃ aus NCO(CH₂)ₙ₄NHR₆, CHOCONH(CH₂)ₙ₅NHR₆ oder CHSCON(CH₂)ₙ₅NHR₆ ausgewählt ist,
wobei n₄ eine ganze Zahl ist, die aus 2 bis 10 ausgewählt ist,
wobei n₅ eine ganze Zahl ist, die aus 2 bis 10 ausgewählt ist,
und wobei R₆ aus H oder Methoxypolyethylenglycolderivaten ausgewählt ist.

2. EPO-mimetisches Peptidderivat nach Anspruch 1 und seine pharmazeutisch akzeptablen Salze, wobei die Aminosäuresequenz von R₁ und R₅ konsistent oder inkonsistent sein kann.

3. EPO-mimetisches Peptidderivat nach einem der Ansprüche 1 bis 2 und seine pharmazeutisch akzeptablen Salze, wobei der N-Terminus von R₁ und R₅ acetylisiert ist.

4. EPO-mimetisches Peptidderivat nach einem der Ansprüche 1 bis 3 und seine pharmazeutisch akzeptablen Salze, wobei R₁ und R₅ zyklische Peptide sind, die durch Disulfidbindungen zyklisiert sind.

5. EPO-mimetisches Peptidderivat nach einem der Ansprüche 1 bis 4 und seine pharmazeutisch akzeptablen Salze, wobei R₁ und R₅ unabhängig aus zyklischen Peptiden ausgewählt sind, die die folgenden Sequenzen aufweisen:

6. EPO-mimetisches Peptidderivat nach einem der Ansprüche 1 bis 5 und seine pharmazeutisch akzeptablen Salze, wobei das EPO-mimetisches Peptid aus den folgenden Peptiden ausgewählt ist, wobei
n₁ und n₂ 2 sind, R₂ und R₄ -CO sind, R₃ CHOCONH(CH₂)ₙ₅NHR₆ ist, n₅ 2 ist,
n₁ und n₂ 1 sind, R₂ und R_{4 -}CO sind, R₃ NCO(CH₂)ₙ₄NHR₆ ist, n₄ 2 ist,
n₁ und n₂ 2 sind, R₂ und R_{4 -}CH₂ sind, R₃ CHOCONH(CH₂)ₙ₅NHR₆ ist, n₅ 2 ist, oder
n₁ und n₂ 1 sind, R₂ und R₄ -CH₂ sind, R₃ NCO(CH₂)ₙ₄NHR₆ ist, n₄ 2 ist.

7. EPO-mimetisches Peptidderivat nach einem der Ansprüche 1 bis 6 und seine pharmazeutisch akzeptablen Salze, **dadurch gekennzeichnet, dass** R₆ H ist.

8. EPO-mimetisches Peptidderivat nach einem der Ansprüche 1 bis 7 und seine pharmazeutisch akzeptablen Salze, wobei R₆ ein Methoxypolyethylenglycolderivat ist, und wobei das Molekulargewicht des Methoxypolyethylenglycolderivats vorzugsweise aus 5.000 bis 100.000 Dalton ausgewählt ist.

9. EPO-mimetisches Peptidderivat nach Anspruch 8 und seine pharmazeutisch akzeptablen Salze, wobei die Struktur des Methoxypolyethylenglycolderivats aus dem verzweigten oder linearen Typ ausgewählt ist, wobei das Methoxypolyethylenglycolderivat vorzugsweise eine lineare Struktur hat und ein Molekulargewicht von 20.000 Dalton aufweist, oder das Methoxypolyethylenglycolderivat eine lineare Struktur hat und ein Molekulargewicht von 40.000 Dalton aufweist.

10. EPO-mimetisches Peptidderivat nach einem der Ansprüche 1 bis 9 und seine pharmazeutisch akzeptablen Salze, wobei
n₁ und n₂ 2 sind, R₁ und R₅ aus SEQ ID NO: 5 bis SEQ ID NO: 8 ausgewählt sind, R₂ und R₄ aus -CO und -CH₂ ausgewählt sind, R₃ CHOCONH(CH₂)n₅NHR₆ ist, wobei n₅ eine ganze Zahl von 2 bis 10 ist, R₆ ein Methoxypolyethylenglycolderivat mit linearer Struktur und einem Molekulargewicht von 20.000 Dalton ist,
n₁ und n₂ 1 sind, R₁ und R₅ aus SEQ ID NO: 5 bis SEQ ID NO: 8 ausgewählt sind, R₂ und R₄ aus -CO und -CH₂ ausgewählt sind, R₃ aus NCO(CH₂)n₄NHR₆ ausgewählt ist, wobei n₄ eine ganze Zahl von 2 bis 10 ist, R₆ ein Methoxypolyethylenglycolderivat mit linearer Struktur und einem Molekulargewicht von 20.000 Dalton ist,
n₁ und n₂ 2 sind, R₁ und R₅ aus SEQ ID NO: 5 bis SEQ ID NO: 8 ausgewählt sind, R₂ und R₄ aus -CO und -CH₂ ausgewählt sind, R₃ CHOCONH(CH₂)n₅NHR₆ ist, wobei n₅ eine ganze Zahl von 2 bis 10 ist, R₆ ein Methoxypolyethylenglycolderivat mit linearer Struktur und einem Molekulargewicht von 40.000 Dalton ist, oder
n₁ und n₂ 1 sind, R₁ und R₅ aus SEQ ID NO: 5 bis SEQ ID NO: 8 ausgewählt sind, R₂ und R₄ aus -CO und -CH₂ ausgewählt sind, R₃ NCO(CH₂)n₄NHR₆ ist, wobei n₄ eine ganze Zahl von 2 bis 10 ist, R₆ ein Methoxypolyethylenglycolderivat mit linearer Struktur und einem Molekulargewicht von 40.000 Dalton ist.

11. EPO-mimetisches Peptidderivat nach einem der Ansprüche 1 bis 10 und seine pharmazeutisch akzeptablen Salze, wobei das EPO-mimetische Peptidderivat und seine pharmazeutisch akzeptablen Salze folgendes ist:

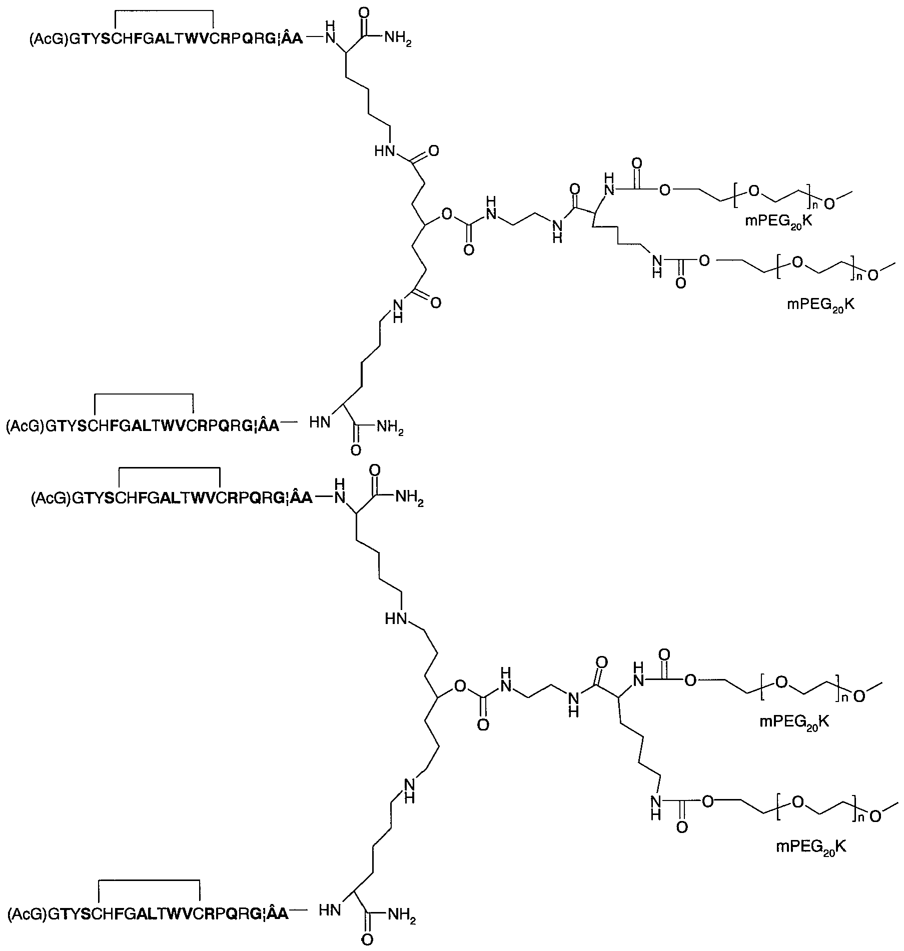

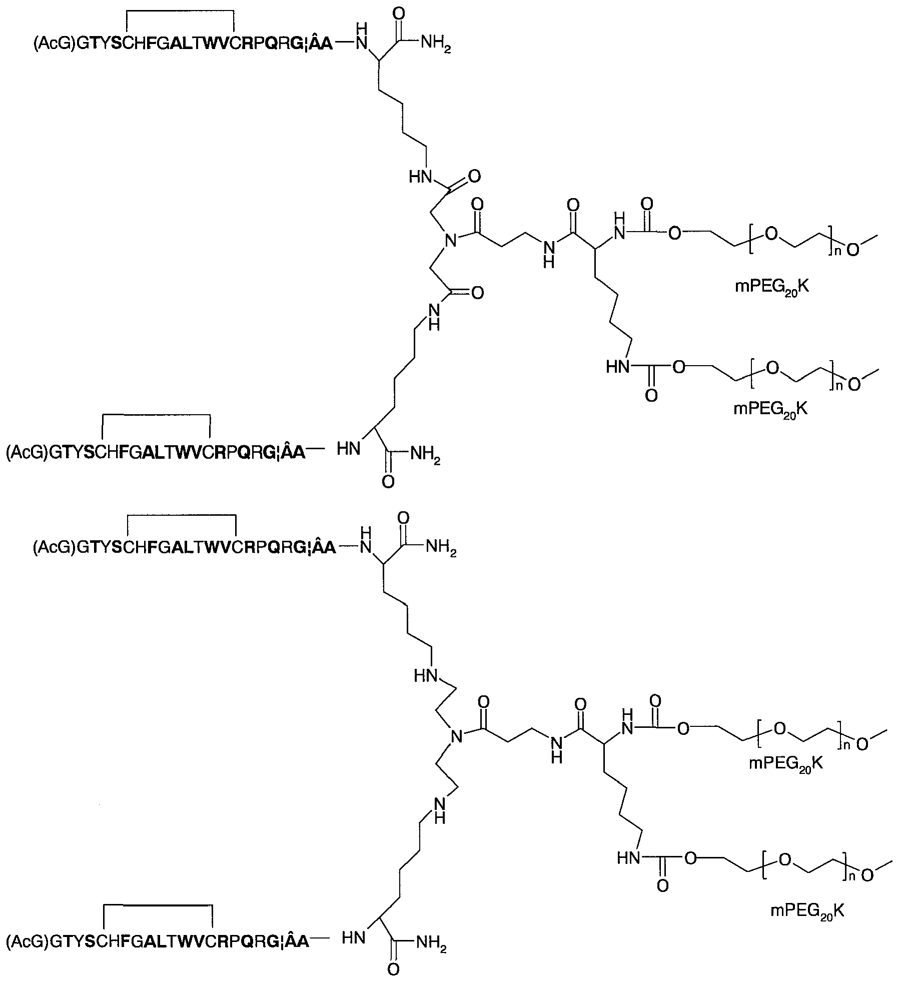

12. Verfahren zum Herstellen eines EPO-mimetischen Peptidderivats nach einem der Ansprüche 1 bis 11 und seine pharmazeutisch akzeptablen Salze davon, umfassend:
(1) Herstellen von R₁H und R₅H, wobei R₁ und R₅ aus zyklischen Peptiden mit den Sequenzen von SEQ ID NOs: 5-8, SEQ ID NOs: 12-20, SEQ ID NOs: 23-26, SEQ ID NOs 29-30 ausgewählt sind,
(2) Zubereiten eines funktionellen kleinen Moleküls der allgemeinen Formel (II)
R₇-CO-(CH₂)ₙ₁-Z₂-(CH₂)ₙ₂-CO-R₈ (II)
wobei n₁ und n₂ ganze Zahlen sind, die unabhängig aus 0 bis 10 ausgewählt sind,
R₇ und R₈ aus OH oder H ausgewählt sind,
Z₂ aus NCO(CH₂)ₙ₇NHR₉, CHOCONH(CH₂)ₙ₈NHR₉ oder CHSCON(CH₂)ₙ₈NHR₉ ausgewählt ist, wobei n₇ eine ganze Zahl ist, die aus 2 bis 10 ausgewählt ist, n₈ eine ganze Zahl ist, die aus 2 bis 10 ausgewählt ist, R₉ aus Boc oder Cbz ausgewählt ist,
(3) Reagieren von R₁ und R₅ mit einem funktionellen kleinen Molekül der Formel (II) durch Amidation oder reduktiver Amination, um die Verbindung der Formel (III) herzustellen
R₁-R₂-(CH₂)ₙ₁-Z₂-(CH₂)ₙ₂-R₄-R₅ (III),
wobei R₂ und R₄ unabhängig aus -CO oder -CH₂ ausgewählt sind,
(4) nach Entfernen von Boc oder Cbz, Ausführen der Amidationsreaktion mit aktivem Methoxypolyethylenglycol.

13. Verfahren zum Herstellen nach Anspruch 12, **dadurch gekennzeichnet, dass** in der allgemeinen Formel (II)
n₁ und n₂ 1 oder 2 sind, R₇ und R₈ OH sind, Z₂ aus NCO(CH₂)ₙ₇NHR₉ oder CHOCONH(CH₂)ₙ₈NHR₉ ausgewählt ist, n₇ eine ganze Zahl ist, die aus 2 bis 10 ausgewählt ist, n₈ eine ganze Zahl ist, die aus 2 bis 10 ausgewählt ist, R₉ Boc ist, oder
n₁ und n₂ 1 oder 2 sind, R₇ und R₈ H sind, Z₂ aus NCO(CH₂)ₙ₇NHR₉ oder CHOCONH(CH₂)ₙ₈NHR₉ ausgewählt ist, n₇ eine ganze Zahl ist, die aus 2 bis 10 ausgewählt ist, n₈ eine ganze Zahl ist, die aus 2 bis 10 ausgewählt ist, R₉ Boc ist.

14. Pharmazeutische Zusammensetzung, umfassend:
(1) eine therapeutisch wirksame Menge des EPO-mimetischen Peptidderivats nach einem der Ansprüche 1 bis 11 oder pharmazeutisch akzeptable Salze davon und
(2) pharmazeutisch akzeptable Trägerstoffe.

15. Verwendung des EPO-mimetischen Peptidderivats nach einem der Ansprüche 1 bis 11 oder pharmazeutisch akzeptable Salze davon oder einer pharmazeutischen Zusammensetzung nach Anspruch 14 in der Zubereitung eines Medikaments zur Behandlung von Erkrankungen, die durch einen niedriges EPO-Spiegel oder durch insuffiziente oder defekte rote Blutkörperchen gekennzeichnet ist.

16. Verwendung nach Anspruch 15, wobei die Erkrankungen, die durch einen niedrigen EPO-Spiegel oder insuffiziente oder defekte rote Blutkörperchen gekennzeichnet sind, eine fortschreitende Niereninsuffizienz oder Dialyse, mit AIDS assoziierte Anämie, Autoimmunerkrankungen oder bösartige Tumoren, zystische Fibrose, Frühgeborenen-Anämie, mit chronisch entzündlichen Erkrankungen assoziierte Anämie, Rückenmarksverletzung, akuter Blutverlust, Alterung und Krebs mit abnormal produzierten roten Blutkörperchen sind.

## Revendications

1. Dérivé de peptide mimétique de l'érythropoïétine (EPO) de formule générale (I) et ses sels pharmaceutiquement acceptables,
R₁-R₂-(CH₂)ₙ₁-R₃-(CH₂)ₙ₂-R₄-R₅ (I)
où R₁, R₅ sont choisis parmi les peptides cycliques ayant les séquences suivantes : où n₁, n₂ sont des entiers choisis indépendamment parmi 0 à 10 ;
où R₂, R₄ sont choisis parmi -CO ou -CH₂ ;
où R₃ est choisi parmi NCO(CH₂)ₙ₄NHR₆, CHOCONH(CH₂)ₙ₅NHR₆, ou CHSCON(CH₂)ₙ₅NHR₆ ;
où n₄ est un entier choisi parmi 2 à 10,
où n₅ est un entier choisi parmi 2 à 10,
et où R₆ est choisi parmi H ou les dérivés de méthoxy polyéthylène glycol.

2. Dérivé de peptide mimétique de l'EPO selon la revendication 1 et ses sels pharmaceutiquement acceptables, où la séquence d'aminoacides de R₁, R₅ pourrait être uniforme ou non uniforme.

3. Dérivé de peptide mimétique de l'EPO selon l'une quelconque des revendications 1 à 2 et ses sels pharmaceutiquement acceptables, où les extrémités N-terminales de R₁, R₅ sont acétylées.

4. Dérivé de peptide mimétique de l'EPO selon l'une quelconque des revendications 1 à 3 et ses sels pharmaceutiquement acceptables, où R₁, R₅ sont un peptide cyclique cyclisé par des liaisons disulfure.

5. Dérivé de peptide mimétique de l'EPO selon l'une quelconque des revendications 1 à 4 et ses sels pharmaceutiquement acceptables, où R₁, R₅ sont choisis séparément parmi les peptides cycliques ayant les séquences suivantes :

6. Dérivé de peptide mimétique de l'EPO selon l'une quelconque des revendications 1 à 5 et ses sels pharmaceutiquement acceptables, où ledit peptide mimétique de l'EPO est choisi parmi le peptide suivant, où
n₁, n₂ sont 2, R₂, R₄ sont -CO, R₃ est CHOCONH(CH₂)ₙ₅NHR₆, n₅ est 2 ;
n₁, n₂ sont 1, R₂, R₄ sont -CO, R₃ est NCO(CH₂)ₙ₄NHR₆, n₄ est 2 ;
n₁, n₂ sont 2, R₂, R₄ sont -CH₂, R₃ est CHOCONH(CH₂)ₙ₅NHR₆, n₅ est 2 ; ou
n₁, n₂ sont 1, R₂, R₄ sont -CH₂, R₃ est NCO(CH₂)ₙ₄NHR₆, n₄ est 2.

7. Dérivé de peptide mimétique de l'EPO selon l'une quelconque des revendications 1 à 6 et ses sels pharmaceutiquement acceptables, **caractérisé en ce que** R₆ est H.

8. Dérivé de peptide mimétique de l'EPO selon l'une quelconque des revendications 1 à 7 et ses sels pharmaceutiquement acceptables, où R₆ est des dérivés de méthoxy polyéthylène glycol et où la masse moléculaire des dérivés de méthoxy polyéthylène glycol est de préférence choisie parmi 5 000 à 100 000 Daltons.

9. Dérivé de peptide mimétique de l'EPO selon la revendication 8 et ses sels pharmaceutiquement acceptables, où la structure des dérivés de méthoxy polyéthylène glycol est choisie parmi le type ramifié ou linéaire, de préférence les dérivés de méthoxy polyéthylène glycol sont de structure linéaire et ont une masse moléculaire de 20 000 daltons, ou les dérivés de méthoxy polyéthylène glycol sont de structure linéaire et ont une masse moléculaire de 40 000 daltons.

10. Dérivé de peptide mimétique de l'EPO selon l'une quelconque des revendications 1 à 9 et ses sels pharmaceutiquement acceptables, où
n₁, n₂ sont 2, R₁, R₅ sont choisis parmi SEQ ID NO:5 à SEQ ID NO:8, R₂, R₄ sont choisis parmi -CO, -CH₂, R₃ est CHOCONH (CH₂) n₅NHR₆, où n₅ est un entier choisi parmi 2 à 10 ; R₆ est un dérivé de méthoxy polyéthylène glycol ayant une structure linéaire et une masse moléculaire de 20 000 daltons ;
n₁, n₂ sont 1, R₁, R₅ sont choisis parmi SEQ ID NO:5 à SEQ ID NO:8, R₂, R₄ sont choisis parmi -CO, -CH₂, R₃ est choisi parmi NCO(CH₂)ₙ₄NHR₆, où n₄ est un entier choisi parmi 2 à 10 ; R₆ est un dérivé de méthoxy polyéthylène glycol de structure linéaire et de masse moléculaire de 20 000 daltons ;
n₁, n₂ sont 2, R₁, R₅ sont choisis parmi SEQ ID NO:5 à SEQ ID NO:8, R₂, R₄ sont choisis parmi -CO, -CH₂, R₃ est CHOCONH(CH₂)ₙ₅NHR₆, où n₅ est un entier choisi parmi 2 à 10 ; R₆ est un dérivé de méthoxy polyéthylène glycol de structure linéaire et de masse moléculaire de 40 000 daltons ; ou
n₁, n₂ sont 1, R₁, R₅ sont choisis parmi SEQ ID NO:5 à SEQ ID NO:8, R₂, R₄ sont choisis parmi -CO, -CH₂, R₃ est NCO(CH₂)ₙ₄NHR₆, où n₄ est un entier choisi parmi 2 à 10 ; R₆ est un dérivé de méthoxy polyéthylène glycol de structure linéaire et de masse moléculaire de 40 000 daltons.

11. Dérivé de peptide mimétique de l'EPO selon l'une quelconque des revendications 1 à 10 et ses sels pharmaceutiquement acceptables, où ledit dérivé de peptide mimétique de l'EPO et ses sels pharmaceutiquement acceptables est

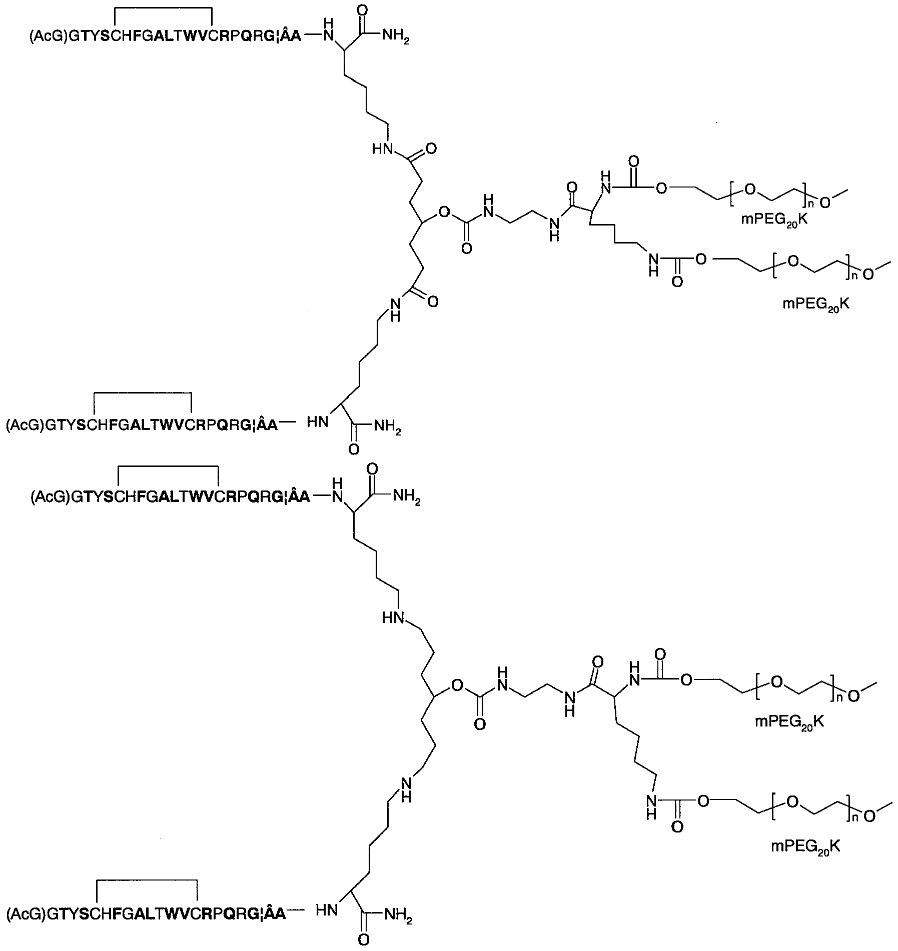

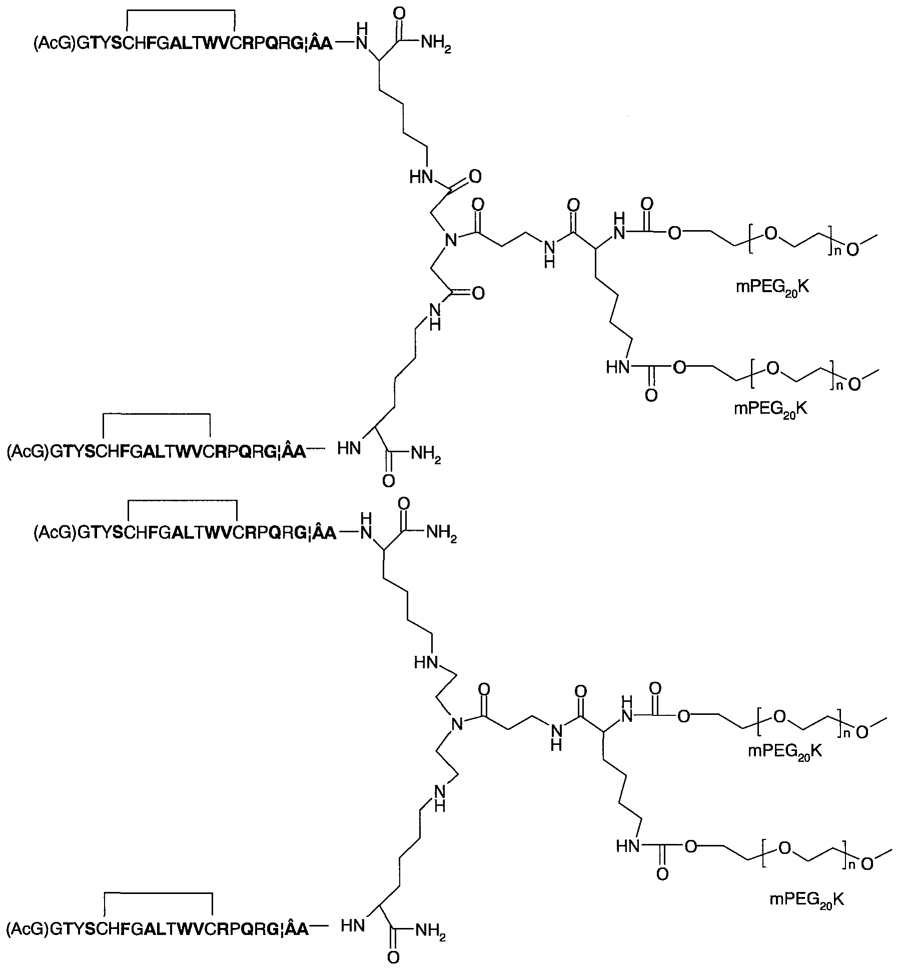

12. Procédé pour la préparation d'un dérivé de peptide mimétique de l'EPO selon l'une quelconque des revendications 1 à 11 et de ses sels pharmaceutiquement acceptables comprenant :
(1) la préparation de R₁H, R₅H, où R₁, R₅ sont choisis parmi les peptides cycliques ayant les séquences de SEQ ID NO:5-8, SEQ ID NO:12-20, SEQ ID NO:23-26, SEQ ID NO:29-30 ;
(2) la préparation d'une petite molécule fonctionnelle de formule générale (II)
R₇-CO-(CH₂)ₙ₁-Z₂-(CH₂)ₙ₂-CO-R₈ (II)
où n₁, n₂ sont des entiers choisis indépendamment parmi 0 à 10 ;
R₇, R₈ sont choisis parmi OH ou H ;
Z₂ est choisi parmi NCO(CH₂)ₙ₇NHR₉, CHOCONH(CH₂)ₙ₈NHR₉, ou CHSCON(CH₂)ₙ₈NHR₉, où n₇ est un entier choisi parmi 2 à 10, n₈ est un entier choisi parmi 2 à 10, R₉ est choisi parmi Boc ou Cbz ;
(3) la réaction de R₁, R₅ avec une petite molécule fonctionnelle de formule (II) par amidation ou amination réductrice pour préparer le composé de formule (III),
R₁-R₂-(CH₂)ₙ₁-Z₂-(CH₂)ₙ₂-R₄-R₅ (III),
où R₂, R₄ sont choisis indépendamment parmi -CO ou -CH₂ ;
(4) après le retrait de Boc ou Cbz, la conduite d'une réaction d'amidation avec un méthoxy polyéthylène glycol actif.

13. Procédé pour la préparation selon la revendication 12, **caractérisé en ce que**, dans ladite formule générale (II)
n₁, n₂ sont 1 ou 2, R₇, R₈ sont OH, Z₂ est choisi parmi NCO(CH₂)ₙ₇NHR₉ ou CHOCONH(CH₂)ₙ₈NHR₉, n₇ est un entier choisi parmi 2 à 10, n₈ est un entier choisi parmi 2 à 10, R₉ est Boc ; ou
n₁, n₂ sont 1 ou 2, R₇, R₈ sont H, Z₂ est choisi parmi NCO(CH₂)ₙ₇NHR₉ ou CHOCONH(CH₂)ₙ₈NHR₉, n₇ est un entier choisi parmi 2 à 10, n₈ est un entier choisi parmi 2 à 10, R₉ est Boc.

14. Composition pharmaceutique comprenant :
(1) une quantité thérapeutiquement efficace du dérivé de peptide mimétique de l'EPO selon l'une quelconque des revendications 1 à 11 ou de sels pharmaceutiquement acceptables de celui-ci, et
(2) des vecteurs pharmaceutiquement acceptables.

15. Utilisation du dérivé de peptide mimétique de l'EPO selon l'une quelconque des revendications 1 à 11 ou de sels pharmaceutiquement acceptables de celui-ci ou d'une composition pharmaceutique selon la revendication 14 dans la préparation d'un médicament pour le traitement de troubles **caractérisés par** un faible niveau d'EPO ou un groupe de globules rouges insuffisants ou défectueux.

16. Utilisation selon la revendication 15, où lesdits troubles **caractérisés par** un faible niveau d'EPO ou un groupe de globules rouges insuffisants ou défectueux sont l'insuffisance rénale avancée ou la dialyse; l'anémie liée au SIDA, les maladies autoimmunes ou une tumeur maligne ; la fibrose kystique ; l'anémie prématurée précoce ; l'anémie liée à des maladies inflammatoires chroniques ; une lésion de la moelle épinière ; une perte de sang aiguë ; le vieillissement et le cancer avec production de globules rouges anormaux.


### sequence Listings

<110> Jiangsu Hansoh Pharmaceutical Co., LTD.
<120> AN ERYTHROPOIETIN MIMETIC PEPTIDE DERIVATIVES AND ITS PHARMACEUTICAL
   SALT, THE PREPARATION AND USES THEREOF
<130> 78017CPCT
<150> CN200710198751.9
   <151> 2007-12-12
<160> 30
<170> PatentIn version 3.4
<210> 1
   <211> 22
   <212> PRT
   <213> Artificial sequence
<220>
   <221> Acetylation
   <222> (1)..(1)
<220>
   <221> Amide
   <222> (6)..(15)
<400> 1
   Gly Gly Leu Tyr Ala Asp His Tyr Gly Pro Ile Thr Trp Val Lys Gln
   1               5                   10                  15
   Pro Leu Arg Gly Gly Lys
               20
<210> 2
   <211> 22
   <212> PRT
   <213> Artificial Sequence
<220>
   <221> Acetylation
   <222> (1)..(1)
<220>
   <221> Amide
   <222> (6)..(15)
<220>
   <221> MISC_FEATURE
   <222> (15)..(15)
   <223> Xaa=orn
<400> 2
   Gly Gly Leu Tyr Ala Asp His Tyr Gly Pro Ile Thr Trp Val Xaa Gln
   1               5                   10                  15
   Pro Leu Arg Gly Gly Lys
               20
<210> 3
   <211> 22
   <212> PRT
   <213> artificial sequence
<220>
   <221> acetylation
   <222> (1)..(1)
<220>
   <221> amide
   <222> (6)..(15)
<400> 3
   Gly Gly Leu Tyr Ala Lys His Tyr Gly Pro Ile Thr Trp Val Asp Gln
   1 5 10 15
   Pro Leu Arg Gly Gly Lys
   20
<210> 4
   <211> 22
   <212> PRT
   <213> artificial sequence
<220>
   <221> acetylation
   <222> (1)..(1)
<220>
   <221> amide
   <222> (6)..(15)
<220>
   <221> MISC_FEATURE
   <222> (6)..(6)
   <223> Xaa=Orn
<400> 4
   Gly Gly Leu Tyr Ala Xaa His Tyr Gly Pro Ile Thr Trp Val Asp Gln
   1 5 10 15
   Pro Leu Arg Gly Gly Lys
   20
<210> 5
   <211> 22
   <212> PRT
   <213> artificial sequence
<220>
   <221> acetylation
   <222> (1)..(1)
<220>
   <221> Disulfide
   <222> (6)..(15)
<220>
   <221> MISC_FEATURE
   <222> (21)..(21)
   <223> Xaa=bAla
<400> 5
   Gly Gly Thr Tyr Ser Cys His Phe Gly Pro Leu Thr Trp Val Cys Arg
   1               5                   10                  15
   Pro Gln Arg Gly Xaa Lys
               20
<210> 6
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (7, 21)
   <223> No. 7 Xaa = Nle; No. 21 Xaa = bAla
<400> 6
   Gly Gly Thr Tyr Ser Cys Xaa Phe Gly Pro Leu Thr Trp Val Cys Arg
   1               5                   10                  15
   Pro Gln Arg Gly Xaa Lys
               20
<210> 7
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 7
   Gly Gly Thr Tyr Ser Cys His Phe Gly Ser Leu Thr Trp Val Cys Arg
   1               5                   10                  15
   Pro Gln Arg Gly Xaa Lys
   20
<210> 8
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 8
   Gly Gly Thr Tyr Ser Cys His Phe Gly Ala Leu Thr Trp Val Cys Arg
   1 5 10 15
   Pro Gln Arg Gly Xaa Lys
   20
<210> 9
   <211> 22
   <212> PRT
   <213> artificial sequence
<220>
   <221> acetylation
   <222> (1)..(1)
<220>
   <221> amide
   <222> (6)..(15)
<400> 9
   Gly Gly Leu Tyr Ala Asp His Tyr Gly Pro Met Thr Trp Val Lys Gln
   1 5 10 15
   Pro Leu Arg Gly Gly Lys
   20
<210> 10
   <211> 22
   <212> PRT
   <213> artificial sequence
<220>
   <221> acetylation
   <222> (1)..(1)
<220>
   <221> amide
   <222> (6)..(15)
<220>
   <221> MISC_FEATURE
   <222> (15)..(15)
   <223> Xaa=Orn
<400> 10
   Gly Gly Leu Tyr Ala Asp His Tyr Gly Pro Met Thr Trp Val Xaa Gln
   1               5                   10                  15
   Pro Leu Arg Gly Gly Lys
               20
<210> 11
   <211> 22
   <212> PRT
   <213> artificial sequence
<220>
   <221> acetylation
   <222> (1)..(1)
<220>
   <221> amide
   <222> (6)..(15)
<220>
   <221> MISC_FEATURE
   <222> (6)..(6)
   <223> Xaa=Orn
<400> 11
   Gly Gly Leu Tyr Ala Xaa His Tyr Gly Pro Met Thr Trp Val Asp Gln
   1               5                   10                  15
   Pro Leu Arg Gly Gly Lys
               20
<210> 12
   <211> 22
   <212> PRT
   <213> artificial sequence
<220>
   <221> acetylation
   <222> (1)..(1)
<220>
   <221> amide
   <222> (6)..(15)
<220>
   <221> MISC_FEATURE
   <222> (21)..(21)
   <223> Xaa=bAla
<400> 12
   Gly Gly Thr Tyr Ser Lys His Phe Gly Pro Met Thr Trp Val Asp Arg
   1               5                   10                  15
   Pro Gln Arg Gly Xaa Lys
               20
<210> 13
   <211> 22
   <212> PRT
   <213> artificial sequence
<220>
   <221> acetylation
   <222> (1) .. (1)
<220>
   <221> disulfide
   <222> (6)..(15)
<220>
   <221> MISC_FEATURE
   <222> (21)..(21)
   <223> Xaa=bAla
<400> 13
   Gly Gly Thr Tyr Ser Cys His Phe Gly Pro Ile Thr Trp Val Cys Arg
   1               5                   10                  15
   Pro Gln Arg Gly Xaa Lys
               20
<210> 14
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (15, 21)
   <223> No. 15 Xaa = Homocysteine; No. 21 Xaa = bAla
<400> 14
   Gly Gly Thr Tyr Ser Cys His Phe Gly Pro Met Thr Trp Val Xaa Arg
   1               5                   10                  15
   Pro Gln Arg Gly Xaa Lys
               20
<210> 15
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (15, 21)
   <223> No. 15 Xaa = Homocysteine; No. 21 Xaa = bAla
<400> 15
   Gly Gly Thr Tyr Ser Cys His Phe Gly Pro Ile Thr Trp Val Xaa Arg
   1               5                   10                  15
   Pro Gln Arg Gly Xaa Lys
               20
<210> 16
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (7, 15, 21)
   <223> No. 7 Xaa = Nle; No. 15 Xaa = Homocysteine; No. 21 Xaa = bAla
<400> 16
   Gly Gly Thr Tyr Ser Cys Xaa Phe Gly Pro Met Thr Trp Val Xaa Arg
   1 5 10 15
   Pro Gln Arg Gly Xaa Lys
   20
<210> 17
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (7, 21)
   <223> No. 7 Xaa = Nle; No. 21 Xaa = bAla
<400> 17
   Gly Gly Thr Tyr Ser Cys Xaa Phe Gly Pro Ile Thr Trp Val Cys Arg
   1 5 10 15
   Pro Gln Arg Gly Xaa Lys
   20
<210> 18
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 18
   Gly Gly Thr Tyr Ser Cys His Phe Gly Pro Leu Thr Trp Val Cys Arg
   1               5                   10                  15
   Pro Gln Arg Gly Xaa Lys
               20
<210> 19
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 19
   Gly Gly Thr Tyr Ser Cys His Phe Gly Ser Ile Thr Trp Val Cys Arg
   1               5                   10                  15
   Pro Gln Arg Gly Xaa Lys
               20
<210> 20
   <211> 22
   <212> PRT
   <213> artificial sequence
<220>
   <221> acetylation
   <222> (1)..(1)
<220>
   <221> amide
   <222> (6)..(15)
<220>
   <221> MISC_FEATURE
   <222> (21)..(21)
   <223> Xaa=bAla
<400> 20
   Gly Gly Thr Tyr Ser Lys His Phe Gly Ser Met Thr Trp Val Glu Arg
   1 5 10 15
   Pro Gln Arg Gly Xaa Lys
   20
<210> 21
   <211> 22
   <212> PRT
   <213> artificial sequence
<220>
   <221> acetylation
   <222> (1)..(1)
<220>
   <221> disulfide
   <222> (6)..(15)
<400> 21
   Gly Gly Thr Tyr Arg Cys Ser Met Gly Pro Met Thr Trp Val Cys Leu
   1 5 10 15
   Pro Met Ala Gly Gly Lys
   20
<210> 22
   <211> 22
   <212> PRT
   <213> artificial sequence
<220> SEQ.txt
   <221> acetylation
   <222> (1)..(1)
<220>
   <221> disulfide
   <222> (6)..(15)
<400> 22
   Gly Gly Thr Tyr Arg Cys Ser Met Gly Pro Leu Thr Trp Val Cys Leu
   1 5 10 15
   Pro Met Ala Gly Gly Lys
   20
<210> 23
   <211> 22
   <212> PRT
   <213> artifificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 23
   Gly Gly Thr Tyr Ser Cys His Phe Gly Ala Met Thr Trp Val Cys Arg
   1 5 10 15
   Pro Gln Arg Gly Xaa Lys
   20
<210> 24
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 24
   Gly Gly Thr Tyr Ser Cys His Phe Gly Ala Ile Thr Trp Val Cys Arg
   1 5 10 15
   Pro Gln Arg Gly Xaa Lys
   20
<210> 25
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 25
   Gly Gly Thr Tyr Ser Cys His Phe Gly Pro Ile Thr Trp Val Cys Arg
   1               5                   10                  15
   Pro Gln Arg Gly Xaa Lys
               20
<210> 26
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 26
   Gly Gly Thr Tyr Ser Cys His Phe Gly Pro Leu Thr Trp Val Cys Arg
   1               5                   10                  15
   Pro Gln Arg Gly Xaa Lys
               20
<210> 27
   <211> 22
   <212> PRT
   <213> artificial sequence
<220>
   <221> zcetylation
   <222> (1)..(1)
<220>
   <221> disulfide
   <222> (6)..(15)
<400> 27
   Gly Gly Met Tyr Ser Cys Arg Met Gly Pro Met Thr Trp Val Cys Gly
   1 5 10 15
   Pro Ser Arg Gly Gly Lys
   20
<210> 28
   <211> 22
   <212> PRT
   <213> artificial sequence
<220>
   <221> acetylation
   <222> (1).. (1)
<220>
   <221> disulfide
   <222> (6)..(15)
<400> 28
   Gly Gly Met Tyr Ser Cys Arg Met Gly Pro Leu Thr Trp Val Cys Gly
   1 5 10 15
   Pro Ser Arg Gly Gly Lys
   20
<210> 29
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (21)..(21)
   <223> Xaa=bAla
<400> 29
   Gly Gly Thr Tyr Ser Cys His Phe Gly Pro Leu Thr Trp Val Xaa Arg
   1 5 10 15
   Pro Gln Arg Gly Xaa Lys
   20
<210> 30
   <211> 22
   <212> PRT
   <213> artificial sequence
<220>
   <221> acetylation
   <222> (1)..(1)
<220>
   <221> disulfide
   <222> (6)..(15)
<220>
   <221> MISC_FEATURE
   <222> (6, 21)
   <223> No. 6 Xaa = Homocysteine; No. 21 Xaa = bAla
<400> 30
   Gly Gly Thr Tyr Ser Xaa His Phe Gly Pro Leu Thr Trp Val Cys Arg
   1               5                   10                  15
   Pro Gln Arg Gly Xaa Lys
               20